# EUROPEAN PATENT SPECIFICATION

(11) **EP 4 014 890 B1**
(45) Date of publication and mention of the grant of the patent: **28.01.2026**
(21) Application number: 20854338.9
(22) Date of filing: 27.07.2020
(51) Int. Cl.: A61B 8/14

(54) **ULTRASONIC DIAGNOSTIC APPARATUS AND CONTROL METHOD FOR ULTRASONIC DIAGNOSTIC APPARATUS**
ULTRASCHALLDIAGNOSEVORRICHTUNG UND STEUERUNGSVERFAHREN FÜR EINE ULTRASCHALLDIAGNOSEVORRICHTUNG
APPAREIL DE DIAGNOSTIC À ULTRASONS ET PROCÉDÉ DE COMMANDE D'UN APPAREIL DE DIAGNOSTIC À ULTRASONS

(30) Priority: 16.08.2019 JP 2019149478
(43) Date of publication of application: 22.06.2022
(73) Proprietor: FUJIFILM Corporation, Tokyo 106-8620 (JP)
(72) Inventor: MATSUMOTO Tsuyoshi, Ashigarakami-gun, Kanagawa 258-8538 (JP)
(74) Representative: Dehns Germany Partnerschaft mbB
(86) International application number: PCT/JP2020/028660
(87) International publication number: WO 2021/033491

(56) References cited:
- JP-A- 2003 126 093
- JP-A- 2005 253 742
- JP-A- 2009 045 427
- JP-A- 2013 116 263
- JP-A- 2013 526 959
- JP-A- 2014 221 161
- JP-A- 2015 198 888
- JP-A- H09 276 278
- US-A1- 2013 102 889
- US-A1- 2014 107 475

## Description

### BACKGROUND OF THE INVENTION

### 1. Field of the Invention

The present invention relates to an ultrasound diagnostic apparatus, and a control method of the ultrasound diagnostic apparatus which display a blood vessel of a subject into which an insertion object is to be inserted, on an ultrasound image.

### 2. Description of the Related Art

In the related art, an ultrasound diagnostic apparatus has been known as an apparatus for obtaining an image of the inside of a subject. The ultrasound diagnostic apparatus generally comprises an ultrasound probe comprising a transducer array in which a plurality of ultrasonic transducers are arranged. In a state where the ultrasound probe is in contact with the body surface of the subject, an ultrasound beam is transmitted toward the inside of the subject from the transducer array and an ultrasound echo from the subject is received by the transducer array so that an electric signal corresponding to the ultrasound echo is acquired. Further, the ultrasound diagnostic apparatus electrically processes the obtained electric signal to generate an ultrasound image of the corresponding site of the subject.

By the way, a procedure of inserting an insertion object such as a so-called puncture needle and a catheter into the blood vessel of the subject while observing the inside of the subject using the ultrasound diagnostic apparatus is known. In a case where such a procedure is performed, normally, by using the ultrasound diagnostic apparatus that can measure a distance between any two points in the ultrasound image as disclosed in JP2010-269018A, a user such as a doctor and a nurse manually measures a diameter of the blood vessel of the subject and a depth from the body surface of the subject in advance, and determines whether the insertion object that the user is trying to insert into the subject can be inserted into the blood vessel of the subject on the basis of the measurement result, in many cases. US 2014/107475 A1discloses an ultrasound diagnostic apparatus that displays a blood vessel of a subject into which an insertion object is to be inserted, on an ultrasound image, the ultrasound diagnostic apparatus comprising:
a. a transducer array;
b. an imaging unit that causes the transducer array to transmit an ultrasound beam toward the subject, and receives an ultrasound echo from the subject to acquire the ultrasound image;
c. a display device that displays the ultrasound image acquired by the imaging unit;
d. a standard information acquisition unit that acquires standard information including standards relating to at least one type of vascular insertion object;
e. a type specifying unit that specifies a type of the insertion object that a user is trying to insert into the blood vessel of the subject;
f. a blood vessel extraction unit that extracts the blood vessel by analyzing the ultrasound image acquired by the imaging unit;
g. an insertability determination unit that determines insertability of the insertion object with respect to the blood vessel extracted by the blood vessel extraction unit, on the basis of the standard information acquired by the standard information acquisition unit and the type of the insertion object specified by the type specifying unit; and
h. a notification unit that notifies of a determination result by the insertability determination unit.

### SUMMARY OF THE INVENTION

However, measuring the diameter and depth of the blood vessel of the subject by using the ultrasound diagnostic apparatus as disclosed in JP2010-269018A and determining whether the insertion object that the user is trying to insert into the subject can be inserted into the blood vessel of the subject on the basis of the measurement result are multi processes and are complicated for the user, in many cases. Further, depending on the position of the blood vessel in the subject and the type of the insertion object, the insertion object can be inserted into the blood vessel in the subject only by properly tilting the insertion object with respect to the subject, and in particular, it may be difficult for a user with a low skill level to accurately determine which insertion object is an insertion object that can be inserted into the blood vessel of the subject. Further, depending on the location such as the country, hospital, and medical department where the ultrasound diagnosis is performed, the type of the recommended insertion object, the incidence angle of the insertion object recommended in a case where the insertion object is inserted into the subject, and the like are often different, and it may be difficult for the user to determine whether the insertion object that the user is trying to insert into the subject can be inserted into the blood vessel.

The present invention has been made in order to solve such a problem in the related art, and an object thereof is to provide an ultrasound diagnostic apparatus and a control method of the ultrasound diagnostic apparatus which allow the user to easily grasp whether the insertion object that the user is trying to insert into the subject can be inserted into the blood vessel of the subject.

According to an aspect of the invention, an ultrasound diagnostic apparatus according to claim 1 is provided.

The ultrasound diagnostic apparatus may further comprise an input device for the user to perform an input operation; and a type acceptance unit that accepts one type of vascular insertion object selected by the user through the input device on the basis of the at least one type of the vascular insertion object included in the standard information acquired by the standard information acquisition unit, in which the type specifying unit may specify the type of the insertion object on the basis of the one type of the vascular insertion object accepted by the type acceptance unit.

In this case, it is preferable that the standard information acquired by the standard information acquisition unit and the type of the insertion object specified by the type specifying unit are displayed on the display device.

Further, it is preferable that the standard information acquired by the standard information acquisition unit and the type of the insertion object specified by the type specifying unit can be modified by the user through the input device.

The ultrasound diagnostic apparatus may further comprise a type information reader that reads type information from a type information holder attached to the insertion object, in which the type specifying unit may specify the type of the insertion object on the basis of the type information read by the type information reader.

The ultrasound diagnostic apparatus may further comprise a camera unit that acquires an image of the insertion object by imaging the insertion object; and an insertion object recognition unit that recognizes the insertion object on the basis of the image of the insertion object acquired by the camera unit, in which the type specifying unit may specify the type of the insertion object on the basis of the insertion object recognized by the insertion object recognition unit.

The ultrasound diagnostic apparatus may further comprise an insertion object recognition unit that recognizes the insertion object on the basis of the ultrasound image acquired by the imaging unit, in which the type specifying unit may specify the type of the insertion object on the basis of the insertion object recognized by the insertion object recognition unit.

Further, it is preferable that the standard information acquired by the standard information acquisition unit and the type of the insertion object specified by the type specifying unit are displayed on the display device.

In this case, it is preferable that the ultrasound diagnostic apparatus further comprises an input device for the user to perform an input operation, and the standard information acquired by the standard information acquisition unit and the type of the insertion object specified by the type specifying unit can be modified by the user through the input device.

Further, it is preferable that the notification unit highlights the blood vessel determined by the insertability determination unit that the insertion object can be inserted, on the display device.

In this case, the insertion object may have a color code assigned according to the standard information, and the notification unit may highlight the blood vessel with the same color as the color code of the insertion object.

Further, the ultrasound image may include a plurality of blood vessels extracted by the blood vessel extraction unit, the insertability determination unit may determine the insertability of the insertion object with respect to each of the plurality of blood vessels, and the notification unit may display, among the plurality of blood vessels, the blood vessel determined by the insertability determination unit that the insertion object can be inserted and the blood vessel determined by the insertability determination unit that the insertion object cannot be inserted, on the display device in a distinguishable manner.

In this case, the ultrasound diagnostic apparatus may further comprise an insertion angle calculation unit that calculates an insertion angle of the insertion object by analyzing the ultrasound image acquired by the imaging unit, in which the insertability determination unit may determine the insertability of the insertion object with respect to each of the plurality of blood vessels on the basis of the insertion angle calculated by the insertion angle calculation unit, and the notification unit may highlight, among the plurality of blood vessels, the blood vessel determined by the insertability determination unit that the insertion object can be inserted, on the display device.

The ultrasound diagnostic apparatus may further comprise a standard information saving memory that saves a plurality of pieces of the standard information, in which the standard information acquisition unit may acquire one piece of the standard information among the plurality of pieces of standard information saved in the standard information saving memory.

Further, the ultrasound diagnostic apparatus may be connected to an external server via a network, and the standard information acquisition unit may acquire the standard information via the server.

In this case, the ultrasound diagnostic apparatus may further comprise a GPS sensor that measures positional information of the ultrasound diagnostic apparatus, in which the standard information acquisition unit may acquire the standard information corresponding to the positional information measured by the GPS sensor.

According to an aspect of the invention, a *control method of an ultrasound diagnostic apparatus according to claim 17 is provided.

### BRIEF DESCRIPTION OF THE DRAWINGS

Fig. 1 is a block diagram illustrating a configuration of an ultrasound diagnostic apparatus according to a first embodiment of the present invention.
Fig. 2 is a block diagram illustrating an internal configuration of a transmission and reception circuit in the first embodiment of the present invention.
Fig. 3 is a block diagram illustrating an internal configuration of an image generation unit in the first embodiment of the present invention.
Fig. 4 is a diagram schematically illustrating an example of an ultrasound image in which a blood vessel is depicted in the first embodiment of the present invention.
Fig. 5 is a diagram illustrating an example of standard information in the first embodiment of the present invention.
Fig. 6 is a flowchart illustrating an operation of the ultrasound diagnostic apparatus according to the first embodiment of the present invention.
Fig. 7 is a diagrams schematically illustrating an example of an ultrasound image in a case where a gel layer is applied to a body surface of a subject in the first embodiment of the present invention.
Fig. 8 is a block diagram illustrating a configuration of an ultrasound diagnostic apparatus according to a second embodiment of the present invention.
Fig. 9 is a block diagram illustrating a configuration of an ultrasound diagnostic apparatus according to a third embodiment of the present invention.
Fig. 10 is a block diagram illustrating a configuration of an ultrasound diagnostic apparatus according to a fourth embodiment of the present invention.
Fig. 11 is a diagram schematically illustrating an example of an ultrasound image in which a blood vessel and an insertion object are depicted in the fourth embodiment of the present invention.
Fig. 12 is a block diagram illustrating a configuration of an ultrasound diagnostic apparatus according to a fifth embodiment of the present invention.
Fig. 13 is a diagram illustrating an ultrasound diagnostic apparatus according to a sixth embodiment of the present invention and a server connected to the ultrasound diagnostic apparatus via a network.
Fig. 14 is a block diagram illustrating a configuration of an ultrasound diagnostic apparatus according to a modification example of the sixth embodiment of the present invention.

### DESCRIPTION OF THE PREFERRED EMBODIMENTS

Hereinafter, embodiments of the invention will be described with reference to the accompanying drawings.

The description of configuration requirements described below is given on the basis of the representative embodiment of the present invention, but the present invention is not limited to such an embodiment.

In the present specification, a numerical range represented using "to" means a range including the numerical values before and after "to" as a lower limit value and an upper limit value.

In the present specification, the terms "same" and "identical" include an error range generally allowed in the technical field.

### First Embodiment

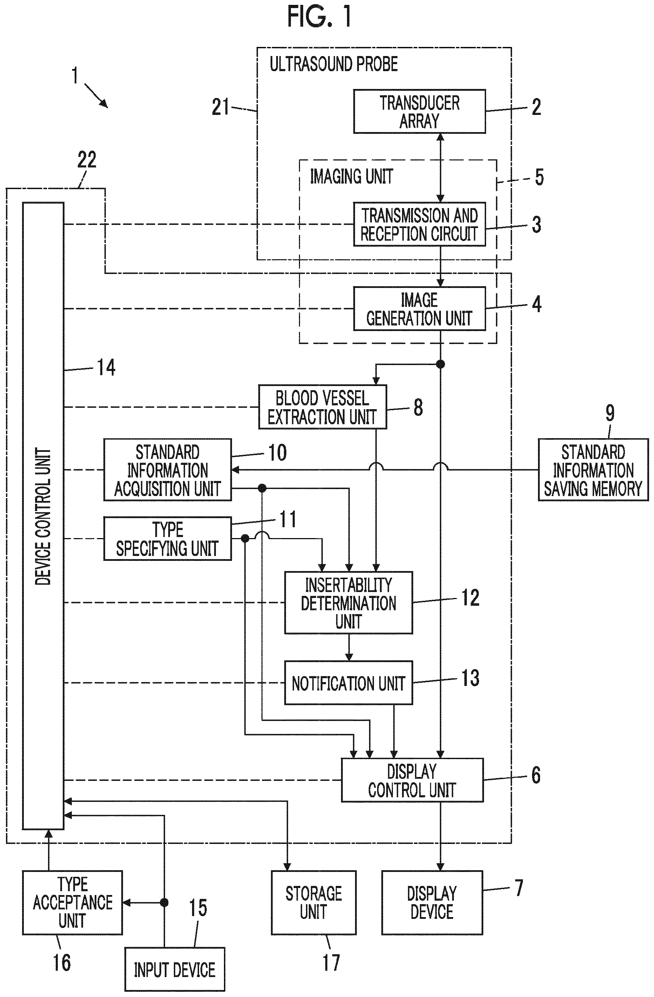

Fig. 1 illustrates a configuration of an ultrasound diagnostic apparatus 1 according to a first embodiment of the present invention. The ultrasound diagnostic apparatus 1 comprises a transducer array 2, and a transmission and reception circuit 3, an image generation unit 4, a display control unit 6, and a display device 7 are sequentially connected to the transducer array 2. Here, the transmission and reception circuit 3 and the image generation unit 4 constitute an imaging unit 5. A blood vessel extraction unit 8 is connected to the image generation unit 4. The ultrasound diagnostic apparatus 1 comprises a standard information saving memory 9, a standard information acquisition unit 10 is connected to the standard information saving memory 9. Further, the ultrasound diagnostic apparatus 1 comprises a type specifying unit 11. The standard information acquisition unit 10 and the type specifying unit 11 are connected to the display control unit 6. The blood vessel extraction unit 8, the standard information acquisition unit 10, and the type specifying unit 11 are connected to an insertability determination unit 12, and the insertability determination unit 12 is connected to a notification unit 13. The notification unit 13 is connected to the display control unit 6.

Further, a device control unit 14 is connected to the transmission and reception circuit 3, the image generation unit 4, the display control unit 6, the blood vessel extraction unit 8, the standard information acquisition unit 10, the type specifying unit 11, the insertability determination unit 12, and the notification unit 13. An input device 15, a type acceptance unit 16, and a storage unit 17 are connected to the device control unit 14. The input device 15 is connected to the type acceptance unit 16. The device control unit 14 and the storage unit 17 are connected so as to exchange information bidirectionally.

The transducer array 2 and the transmission and reception circuit 3 are included in an ultrasound probe 21. Further, the image generation unit 4, the display control unit 6, the blood vessel extraction unit 8, the standard information acquisition unit 10, the type specifying unit 11, the insertability determination unit 12, and the notification unit 13 constitute a processor 22 for the ultrasound diagnostic apparatus 1.

The transducer array 2 of the ultrasound probe 21 illustrated in Fig. 1 has a plurality of transducers arranged in a one-dimensional or two-dimensional manner. According to a drive signal supplied from the transmission and reception circuit 3, each of the transducers transmits an ultrasonic wave and receives an ultrasound echo from a subject to output a signal based on the ultrasound echo. For example, each transducer is configured by forming electrodes at both ends of a piezoelectric body consisting of piezoelectric ceramic represented by lead zirconate titanate (PZT), a polymer piezoelectric element represented by poly vinylidene di fluoride (PVDF), piezoelectric single crystal represented by lead magnesium niobate-lead titanate (PMN-PT), or the like.

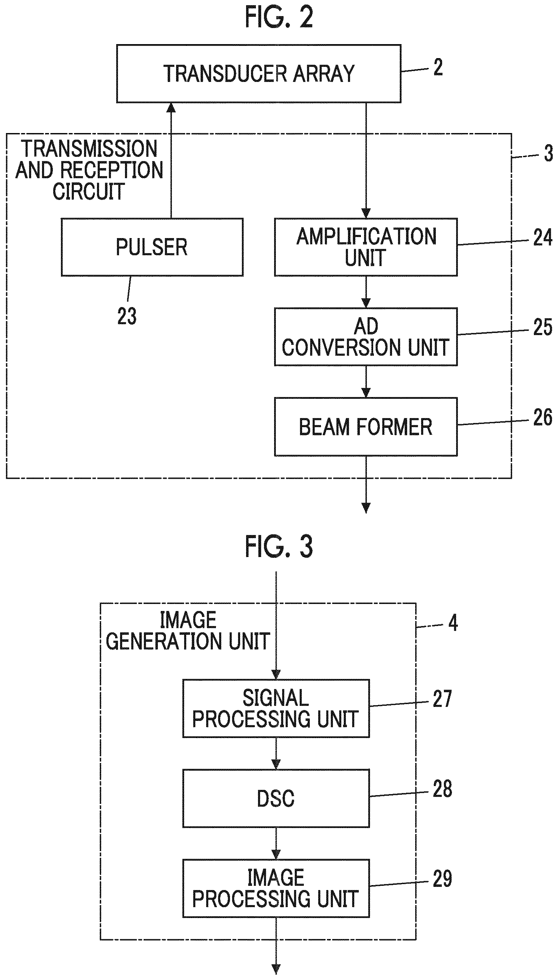

The transmission and reception circuit 3 causes the transducer array 2 to transmit the ultrasonic wave and generates a sound ray signal on the basis of a reception signal acquired by the transducer array 2, under the control of the device control unit 14. As illustrated in Fig. 2, the transmission and reception circuit 3 has a pulser 23 connected to the transducer array 2, and an amplification unit 24, an analog digital (AD) conversion unit 25, and a beam former 26 that are sequentially connected in series from the transducer array 2.

The pulser 23 includes, for example, a plurality of pulse generators, and the pulser 23 adjusts the amount of delay of each drive signal so that ultrasonic waves transmitted from the plurality of transducers of the transducer array 2 form an ultrasound beam on the basis of a transmission delay pattern selected according to the control signal from the device control unit 14, and supplies the obtained signals to the plurality of transducers. Thus, in a case where a pulsed or continuous-wave voltage is applied to the electrodes of the transducers of the transducer array 2, the piezoelectric body expands and contracts to generate pulsed or continuous-wave ultrasonic waves from each transducer. From the combined wave of these ultrasonic waves, an ultrasound beam is formed.

The transmitted ultrasound beam is reflected by a target, for example, a site of the subject, and propagates toward the transducer array 2 of the ultrasound probe 21. Each transducer constituting the transducer array 2 expands and contracts by receiving the ultrasound echo propagating toward the transducer array 2 in this manner, to generate the reception signal that is an electric signal, and outputs the reception signal to the amplification unit 24.

The amplification unit 24 amplifies the signals input from each transducer constituting the transducer array 2, and transmits the amplified signals to the AD conversion unit 25. The AD conversion unit 25 converts the signal transmitted from the amplification unit 24 into digital reception data, and transmits the reception data to the beam former 26. The beam former 26 performs so-called reception focusing processing in which addition is performed by giving delays to respective pieces of the reception data converted by the AD conversion unit 25 according to a sound speed distribution or a sound speed set on the basis of a reception delay pattern selected according to the control signal from the device control unit 14. Through the reception focusing processing, a sound ray signal in which each piece of the reception data converted by the AD conversion unit 25 is phased and added and the focus of the ultrasound echo is narrowed is acquired.

As illustrated in Fig. 3, the image generation unit 4 has a configuration in which a signal processing unit 27, a digital scan converter (DSC) 28, and an image processing unit 29 are sequentially connected in series.

The signal processing unit 27 generates a B-mode image signal, which is tomographic image information regarding tissues inside the subject, by performing, on the sound ray signal generated by the beam former 26 of the transmission and reception circuit 3, correction of the attenuation due to the distance according to the depth of the reflection position of the ultrasonic wave and then performing envelope detection processing.

The DSC 28 converts (raster conversion) the B-mode image signal generated by the signal processing unit 27 into an image signal according to a normal television signal scanning method.

The image processing unit 29 performs various kinds of necessary image processing such as gradation processing on the B-mode image signal input from the DSC 28, and then outputs the B-mode image signal to the display control unit 6 and the blood vessel extraction unit 8. In the following, the B-mode image signal subjected to the image processing by the image processing unit 29 is simply referred to as an ultrasound image.

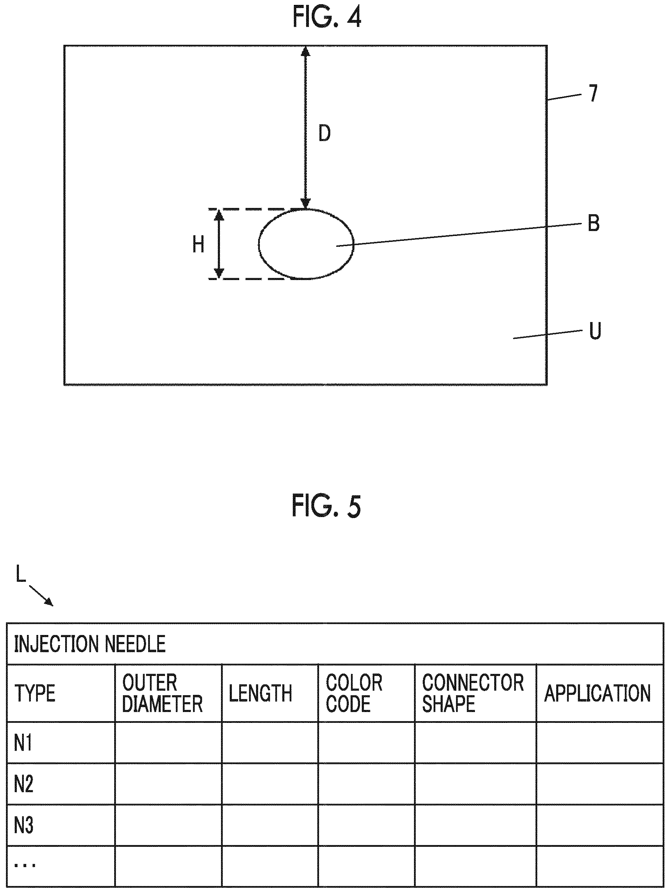

The blood vessel extraction unit 8 extracts a blood vessel B included in an ultrasound image U, for example, as illustrated in Fig. 4 by analyzing the ultrasound image generated by the image generation unit 4. Here, the blood vessel extraction unit 8 can extract the blood vessel B in the ultrasound image U using a known algorithm. For example, the blood vessel extraction unit 8 can store typical pattern data of the blood vessel B in advance as a template, calculate a similarity degree for the pattern data while searching the ultrasound image U using the template, and consider that the blood vessel B is present in a place where the similarity degree is equal to or greater than a threshold value and is the maximum.

Further, for the calculation of the similarity degree, in addition to simple template matching, for example, a machine learning method described in Csurka et al.: Visual Categorization with Bags of Keypoints, Proc. of ECCV Workshop on Statistical Learning in Computer Vision, pp. 59-74 (2004) or a general image recognition method using deep learning described in Krizhevsk et al.: ImageNet Classification with Deep Convolutional Neural Networks, Advances in Neural Information Processing Systems 25, pp.1106-1114 (2012) can be used.

Further, the blood vessel extraction unit 8 can measure a diameter H of the extracted blood vessel B and a depth of the blood vessel B, that is, a distance D from a body surface of the subject to the blood vessel B, as illustrated in Fig. 4, by analyzing the ultrasound image U. Here, the blood vessel extraction unit 8 can measure, for example, a width of the blood vessel B in a depth direction of the ultrasound image U as the diameter H of the blood vessel B. The blood vessel extraction unit 8 can measure the shortest distance from the upper end portion of the ultrasound image U to the blood vessel B as the distance D from the body surface of the subject to the blood vessel B.

In general, a procedure of inserting an insertion object such as a so-called puncture needle and a catheter into the blood vessel of the subj ect while observing the inside of the subject using the ultrasound diagnostic apparatus is known. There are many types of vascular insertion objects used in such a procedure, and, for example, recommended types of vascular insertion objects may differ depending on the location such as the country, hospital, and medical department.

The standard information saving memory 9 saves a plurality of pieces of standard information including standards for a plurality of types of vascular insertion objects each corresponding to the location such as the country, hospital, and medical department. Here, the standard for the vascular insertion object represents features of the vascular insertion object, and can include, for example, the type, outer diameter, length, so-called color code, and connector shape of the vascular insertion object, and the application of the vascular insertion object. Further, for example, as illustrated in Fig. 5, the standard information is a table in which standards for vascular insertion objects are summarized, and includes standards for at least one type of vascular insertion object.

In the example illustrated in Fig. 5, standard information L regarding an injection needle as the vascular insertion object is illustrated, the type, outer diameter, length, color code, connector shape, and application are illustrated as the standards included in the standard information L, and N1, N2, N3, and the like are shown as the types of injection needles. Further, the color code is generally assigned to a puncture needle, a catheter, and the like, and has a color corresponding to the outer diameter of the vascular insertion object for each application of the vascular insertion object. A correspondence relationship between the application and outer diameter of the vascular insertion object and the color code may differ depending on the country. Further, the application includes, for example, applications for a so-called peripherally intravenous catheter (PIVC), a central venous catheter (CVC), a nerve block, and the like.

The standard information acquisition unit 10 acquires one piece of standard information L among the plurality of pieces of standard information L saved in the standard information saving memory 9. For example, in a case where one of the plurality of pieces of standard information L saved in the standard information saving memory 9 is selected by a user's input operation through the input device 15, the standard information acquisition unit 10 can acquire the standard information L selected by the user.

The input device 15 is for the operator to perform an input operation, and can be configured to comprise a keyboard, a mouse, a trackball, a touchpad, a touch panel, and the like.

The type acceptance unit 16 accepts one type of vascular insertion object selected by the user through the input device 15, on the basis of at least one type of vascular insertion object included in the standard information L acquired by the standard information acquisition unit 10.

The type specifying unit 11 specifies the type of the insertion obj ect that the user is trying to insert into the blood vessel B of the subject. For example, the type specifying unit 11 can specify one type of the vascular insertion object accepted by the type acceptance unit 16 according to the user's input operation through the input device 15, as the type of the insertion object that the user is trying to insert into the blood vessel B of the subject.

The insertability determination unit 12 determines the insertability of the insertion object with respect to the blood vessel B extracted by the blood vessel extraction unit 8, on the basis of the standard information L acquired by the standard information acquisition unit 10 and the type of the insertion object specified by the type specifying unit 11. In a case where the length of the insertion object specified by the type specifying unit 11 is acquired with reference to the standard information L and the acquired length of the insertion object is equal to or less than the distance D from the body surface of the subject to the blood vessel B, which is measured by the blood vessel extraction unit 8, the insertability determination unit 12 can determine that the insertion object that the user is trying to insert into the subject cannot be inserted into the blood vessel B of the subject.

Further, in a case where the acquired length of the insertion object is longer than the distance D from the body surface of the subject to the blood vessel B, which is measured by the blood vessel extraction unit 8, the insertability determination unit 12 can determine that the insertion object that the user is trying to insert into the subject can be inserted into the blood vessel B of the subject. However, in general, in a case where the insertion object such as a puncture needle and a catheter is inserted into the subject, the insertion object is inserted into the subject in a state where the insertion object is tilted with respect to the body surface of the subject in many cases, and an insertion angle of the insertion object in this case is determined according to the location such as the country, hospital, and medical department in many cases.

Therefore, in a case where a recommended insertion angle of the vascular insertion object is included in the standard information L, the insertability determination unit 12 can estimate whether or not the insertion object reaches the blood vessel B on the assumption that the insertion object is inserted into the subject at the recommended insertion angle, for example. In this case, for example, the insertability determination unit 12 acquires the length of the insertion object specified by the type specifying unit 11 with reference to the standard information L, and estimates whether or not the insertion object reaches the blood vessel B in a case where it is assumed that the insertion object is inserted into the subject at the recommended insertion angle, on the basis of the distance D from the body surface of the subject to the blood vessel B, which is measured by the blood vessel extraction unit 8, and the acquired length of the insertion object. Then, the insertability determination unit 12 can determine that the insertion object can be inserted into the blood vessel B in a case where it is estimated that the insertion object reaches the blood vessel B, and can determine that the insertion object cannot be inserted into the blood vessel B in a case where it is estimated that the insertion object does not reach the blood vessel B.

Further, for example, the insertability determination unit 12 can determine whether the insertion object can be inserted into the blood vessel B by adding a relationship between the diameter H of the blood vessel B measured by the blood vessel extraction unit 8 and the outer diameter of the insertion object specified by the type specifying unit 11. In this case, in a case where a ratio of the outer diameter of the insertion object to the diameter of the blood vessel B is greater than a threshold value, the insertability determination unit 12 determines that the outer diameter of the insertion object is too large for the diameter of the blood vessel B, and thus can determine that the insertion object cannot be inserted into the blood vessel B even if it is estimated that the insertion object reaches the blood vessel B, for example. Further, in a case where it is estimated that the insertion object reaches the blood vessel B and the ratio of the outer diameter of the insertion object to the diameter of the blood vessel B is equal to or less than the threshold value, the insertability determination unit 12 can determine that the insertion object can be inserted into the blood vessel B.

The notification unit 13 notifies a determination result by the insertability determination unit 12 to the user. The notification unit 13 can notify the determination result by the insertability determination unit 12 to the user by displaying, for example, a message representing that the insertion object can be inserted into the blood vessel B or a message representing that the insertion object cannot be inserted into the blood vessel B on the display device 7.

The display control unit 6 performs predetermined processing on the ultrasound image U generated by the image generation unit 4, the standard information L acquired by the standard information acquisition unit 10, the type of the insertion object specified by the type specifying unit 11, the information representing the notification to the user by the notification unit 13, and the like under the control of the device control unit 14, and displays the processed ultrasound image U, standard information L, type of the insertion object, information, and the like on the display device 7.

The display device 7 is for displaying the ultrasound image U generated by the image generation unit 4, the standard information L acquired by the standard information acquisition unit 10, the type of the insertion object specified by the type specifying unit 11, the information representing the notification to the user by the notification unit 13, and the like under the control of the display control unit 6, and includes a display device such as a liquid crystal display (LCD), or an organic electroluminescence (EL) display.

The device control unit 14 controls each unit of the ultrasound diagnostic apparatus 1 on the basis of a control program and the like stored in advance.

The storage unit 17 stores a control program and the like of the ultrasound diagnostic apparatus 1, and recording media such as a flash memory, a hard disk drive (HDD), a solid state drive (SSD), a flexible disc (FD), a magneto-optical disc (MO disc), a magnetic tape (MT), a random access memory (RAM), a compact disc (CD), a digital versatile disc (DVD), a secure digital card (SD card), and a universal serial bus memory (USB memory), a server, or the like can be used.

The processor 22 having the image generation unit 4, the display control unit 6, the blood vessel extraction unit 8, the standard information acquisition unit 10, the type specifying unit 11, the insertability determination unit 12, the notification unit 13, and the device control unit 14 is configured by a central processing unit (CPU) and a control program for causing the CPU to execute various kinds of processing, but the processor 22 may be configured by using a field programmable gate array (FPGA), a digital signal processor (DSP), an application specific integrated circuit (ASIC), a graphics processing unit (GPU), or other integrated circuits (IC) or may be configured by a combination thereof.

In addition, the image generation unit 4, the display control unit 6, the blood vessel extraction unit 8, the standard information acquisition unit 10, the type specifying unit 11, the insertability determination unit 12, the notification unit 13, and the device control unit 14 of the processor 22 can also be configured by being integrated partially or entirely into one CPU or the like.

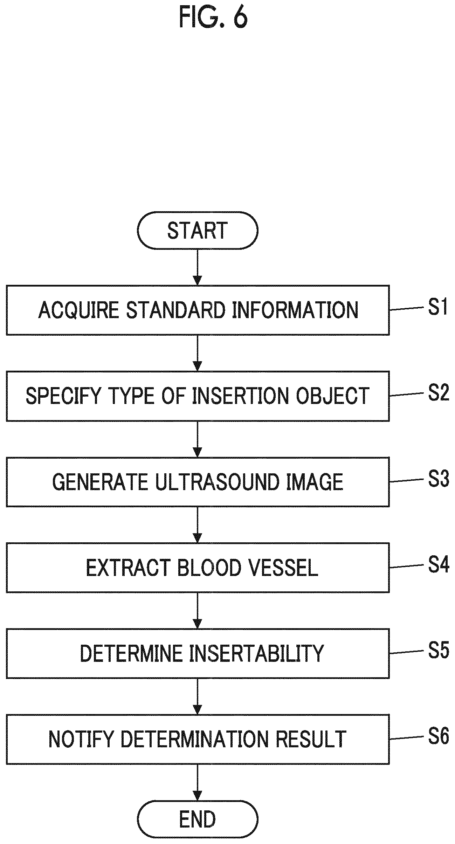

In the following, the operation of the ultrasound diagnostic apparatus 1 of the first embodiment will be described in detail using the flowchart illustrated in Fig. 6.

First, in Step S1, the standard information acquisition unit 10 acquires one piece of standard information L among the plurality of pieces of standard information L as illustrated in Fig. 4, which are saved in the standard information saving memory 9. In this case, for example, with the input of an instruction to select the standard information L by the user through the input device 15 as a trigger, the plurality of pieces of standard information L saved in the standard information saving memory 9 are displayed on the display device 7, and one piece of standard information L among the plurality of pieces of standard information L displayed on the display device 7 can be selected by the user. The standard information acquisition unit 10 can acquire the standard information L selected by the user in this manner.

In the procedure of inserting the insertion object into the subject, since the recommended insertion object differs depending on the location such as the country, hospital, and medical department in some cases, the insertion object corresponding to the location such as the country, hospital, and medical department where the user performs the ultrasound diagnosis can be specified by acquiring one piece of standard information L in this manner.

Next, in Step S2, the type specifying unit 11 specifies the insertion object that the user is trying to insert into the subject, on the basis of the standard information L acquired in Step S1. For example, in a case where the standard information L acquired in Step S1 includes standards for a plurality of types of vascular insertion objects, among the plurality of types of vascular insertion objects included in the standard information L, one type of vascular insertion object is selected by the user through the input device 15, and in a case where the type acceptance unit 16 accepts the one vascular insertion object selected by the user, the type specifying unit 11 can specify the vascular insertion object accepted by the type acceptance unit 16, as the insertion object that the user is trying to insert into the subject. In this case, for example, the standard information L acquired in Step S1 is displayed on the display device 7, and the user can select one of the plurality of vascular insertion objects included in the standard information L displayed on the display device 7, through the input device 15. The type specifying unit 11 displays the specified type of the insertion object on the display device 7.

Further, for example, in a case where the standard information L acquired in Step S1 includes only the standard for one type of vascular insertion object, the one type of vascular insertion object included in the standard information L is selected by the user through the input device 15, and the type acceptance unit 16 accepts the one type of vascular insertion object selected by the user. The type specifying unit 11 can specify the vascular insertion object accepted by the type acceptance unit 16, as the insertion object that the user is trying to insert into the subject.

In Step S3, the ultrasound image U in which at least the blood vessel B of the subject is imaged is generated, and the generated ultrasound image U is displayed on the display device 7. In this case, the ultrasound probe 21 is brought into contact with the body surface of the subject by the user, an ultrasound beam is transmitted into the subject from the plurality of transducers of the transducer array 2 according to the drive signal from the pulser 23 of the transmission and reception circuit 3, and the reception signal is output to the amplification unit 24 of the transmission and reception circuit 3 from each transducer which has received the ultrasound echo from the subject. The reception signal is amplified in the amplification unit 24, is subjected to the AD conversion in the AD conversion unit 25, and is phased and added in the beam former 26, and thereby the sound ray signal is generated. The sound ray signal is subjected to the envelope detection processing by the signal processing unit 27 to become the B-mode image signal in the image generation unit 4, and is output to the display control unit 6 via the DSC 28 and the image processing unit 29, and the ultrasound image U is displayed on the display device 7 under the control of the display control unit 6 as illustrated in Fig. 4.

In Step S4, the blood vessel extraction unit 8 extracts the blood vessel B included in the ultrasound image U by analyzing the ultrasound image U generated in Step S3. In this case, the blood vessel extraction unit 8 can extract the blood vessel B by using, for example, a known algorithm such as template matching, a machine learning method, a general image recognition method using deep learning or the like. Further, the blood vessel extraction unit 8 measures the diameter H of the extracted blood vessel B and the distance D from the body surface of the subject to the blood vessel B, as illustrated in Fig. 4, by analyzing the ultrasound image U.

In Step S5, the insertability determination unit 12 determines whether the insertion object specified in Step S2 can be inserted into the blood vessel B extracted in Step S4, on the basis of the standard information L acquired in Step S1, the type of the insertion object specified in Step S2, and the blood vessel B of the subject extracted in Step S4.

In this case, in a case where the length of the insertion object specified by the type specifying unit 11 is acquired with reference to the standard information L and the acquired length of the insertion object is equal to or less than the distance D from the body surface of the subject to the blood vessel B, which is measured by the blood vessel extraction unit 8, the insertability determination unit 12 can determine that the insertion object that the user is trying to insert into the subject cannot be inserted into the blood vessel B of the subject. Further, in a case where the acquired length of the insertion object is longer than the distance D from the body surface of the subject to the blood vessel B, which is measured by the blood vessel extraction unit 8, the insertability determination unit 12 can determine that the insertion object that the user is trying to insert into the subject can be inserted into the blood vessel B of the subject.

In a case where the recommended insertion angle of the vascular insertion object is included in the standard information L, for example, the insertability determination unit 12 can acquire the length of the insertion object specified by the type specifying unit 11 with reference to the standard information L, and can estimate whether or not the insertion object reaches the blood vessel B in a case where it is assumed that the insertion object is inserted into the subject at the recommended insertion angle, on the basis of the distance D from the body surface of the subject to the blood vessel B, which is measured by the blood vessel extraction unit 8, and the acquired length of the insertion object. Further, the insertability determination unit 12 can determine that the insertion object can be inserted into the blood vessel B in a case where it is estimated that the insertion object reaches the blood vessel B, and can determine that the insertion object cannot be inserted into the blood vessel B in a case where it is estimated that the insertion object does not reach the blood vessel B.

Further, for example, the insertability determination unit 12 can determine whether the insertion object can be inserted into the blood vessel B by adding the relationship between the diameter H of the blood vessel B measured by the blood vessel extraction unit 8 and the outer diameter of the insertion object specified by the type specifying unit 11. In this case, in a case where the ratio of the outer diameter of the insertion object to the diameter of the blood vessel B is greater than the threshold value, the insertability determination unit 12 determines that the outer diameter of the insertion object is too large for the diameter of the blood vessel B, and thus can determine that the insertion object cannot be inserted into the blood vessel B even if it is estimated that the insertion object reaches the blood vessel B, for example. Further, in a case where it is estimated that the insertion object reaches the blood vessel B and the ratio of the outer diameter of the insertion object to the diameter of the blood vessel B is equal to or less than the threshold value, the insertability determination unit 12 can determine that the insertion object can be inserted into the blood vessel B.

In Step S6, the notification unit 13 notifies the determination result of Step S5 to the user. For example, the notification unit 13 can notify the determination result of Step S5 to the user by displaying a message indicating the determination result of Step S5 on the display device 7. For example, in a case where the user has checked the determination result indicating that the insertion object that the user is trying to insert into the subject can be inserted into the blood vessel B of the subject, the user can execute the procedure of inserting the insertion object into the subject. On the other hand, in a case where the user has checked the determination result indicating that the insertion object that the user is trying to insert into the subject cannot be inserted into the blood vessel B of the subject, for example, the insertion object is replaced with a new insertion object. The processing of Step S2 to Step S6 is performed on the basis of the replaced new insertion object, so that it can determined whether the new insertion object can be inserted into the blood vessel B of the subject, and the determination result can be notified to the user by the notification unit 13.

As described above, with the ultrasound diagnostic apparatus 1 according to the first embodiment of the present invention, the insertability determination unit 12 determines whether the insertion object that the user is trying to insert into the subject can be inserted into the blood vessel B of the subject, on the basis of the standard information L acquired by the standard information acquisition unit 10, the type of the insertion object specified by the type specifying unit 11, and the blood vessel B in the ultrasound image U extracted by the blood vessel extraction unit 8, and the notification unit 13 notifies the determination result by the insertability determination unit 12 to the user. Therefore, regardless of the location such as the location such as the country, hospital, and medical department where the ultrasound diagnosis is performed, it is possible for the user to easily grasp whether the insertion object that the user is trying to insert into the subject can be inserted into the blood vessel B of the subject before inserting the insertion object into the subject.

The beam former 26 that performs so-called reception focusing processing is included in the transmission and reception circuit 3, but can be included in the image generation unit 4, for example. Also in this case, similarly to the case where the beam former 26 is included in the transmission and reception circuit 3, the ultrasound image U is generated by the image generation unit 4.

Although the ultrasound probe 21 and the processor 22 are connected to each other, the ultrasound probe 21 and the processor 22 can be connected to each other by so-called wired communication, and can also be connected to each other by so-called wireless communication.

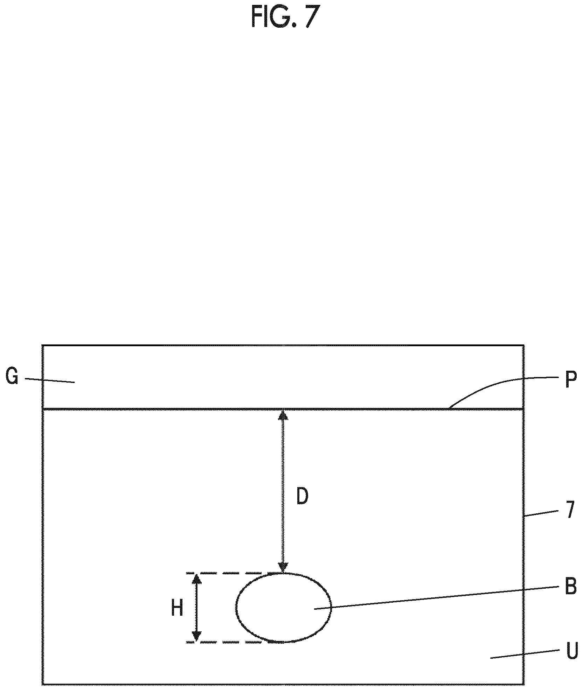

The blood vessel extraction unit 8 measures the distance from the upper end portion of the ultrasound image U to the blood vessel B as the distance D from the body surface of the subject to the blood vessel B, but in a case where the body surface of the subject is depicted in the ultrasound image U, the blood vessel extraction unit 8 can measure the shortest distance from the body surface of the subject depicted in the ultrasound image U to the blood vessel B, as the distance D. For example, in a case where a so-called gel layer is applied to the body surface of the subject in order to reduce the reflection and attenuation of the ultrasonic waves between the ultrasound probe 21 and the body surface of the subject by filling a gap between the ultrasound probe 21 and the body surface of the subject, a gel layer G and a body surface P of the subject are depicted in the ultrasound image U as illustrated in Fig. 7.

In this case, by analyzing the ultrasound image U, the blood vessel extraction unit 8 can recognize the body surface P of the subject, and measure the shortest distance from the recognized body surface P of the subject to the blood vessel B, as the distance D from the body surface P of the subject to the blood vessel B. In this case, the blood vessel extraction unit 8 can recognize the body surface P of the subject by using, for example, a known algorithm such as template matching, a machine learning method, a general image recognition method using deep learning or the like.

Further, the standard information acquisition unit 10 acquires the standard information L selected by the user through the input device 15 among the plurality of pieces of standard information L saved in the standard information saving memory 9, but the standard information L acquired by the standard information acquisition unit 10 can be modified by the user's input operation through the input device 15, for example.

Further, the type specifying unit 11 specifies the vascular insertion object, which is selected by the user through the input device 15 on the basis of at least one vascular insertion object included in the standard information L, as the insertion object that the user is trying to insert into the subject, but the information indicating the type of the insertion object specified by the type specifying unit 11 can also be modified by the user's input operation through the input device 15.

Further, the type specifying unit 11 specifies the type of the insertion object that the user is trying to insert into the subject, according to the user's input operation through the input device 15, but the type specifying unit 11 can specify the type of the insertion object on the basis of the connector shape of the insertion object, for example. For example, although not illustrated, the ultrasound diagnostic apparatus 1 can comprise a connector connection unit that estimates the type of the insertion object to which the connector is connected, by connecting the connector to the insertion object. In this case, the type specifying unit 11 specifies the type of the insertion object that the user is trying to insert into the subject, on the basis of the information indicating the type of the insertion object estimated by the connector connection unit. For example, the type specifying unit 11 can specify the vascular insertion object, which corresponds to the insertion object estimated by the connector connection unit, in the standard information L acquired by the standard information acquisition unit 10, as the insertion object that the user is trying to insert into the subject.

In a case where the insertability determination unit 12 determines that the insertion object that the user is trying to insert into the subject cannot be inserted into the blood vessel B of the subject, the fact is notified to the user by the notification unit 13, but in this case, the notification unit 13 can notify, for example, the fact that it is recommended to change the insertion object that the user is trying to insert into the subject, to the user. In this case, the insertability determination unit 12 can select the type of the vascular insertion object that can be inserted into the blood vessel B of the subject, from the standard information L, and the notification unit 13 can notify the information indicating the type of the vascular insertion object selected by the insertability determination unit 12 to the user.

Further, it has been described that the insertability determination unit 12 determines whether the insertion object can be inserted into the blood vessel B of the subject in a case where it is assumed that the insertion object is inserted into the subject at the recommended insertion angle, on the basis of the standard information L acquired by the standard information acquisition unit 10. However, in a case where it is determined that the insertion object that the user is trying to insert into the subject cannot be inserted into the blood vessel B of the subject, the insertability determination unit 12 can also calculate the insertion angle so that the insertion object can be inserted into the blood vessel B of the subject. In this case, the notification unit 13 can notify the insertion angle calculated by the insertability determination unit 12, to the user.

Further, the notification unit 13 notifies the determination result to the user by displaying the message indicating the determination result by the insertability determination unit 12 on the display device 7, but the method of notifying of the determination result is not limited thereto. For example, in a case where the insertion object that the user is trying to insert into the subject can be inserted into the blood vessel B of the subject, the notification unit 13 highlights the blood vessel B in the ultrasound image U on the display device 7, and in a case where the insertion object that the user is trying to insert into the subject cannot be inserted into the blood vessel B of the subject, the notification unit 13 does not highlight the blood vessel B in the ultrasound image U on the display device 7. Here, highlighting the blood vessel B includes, for example, displaying the blood vessel B by assigning, to the blood vessel B, a color different from that of the other regions in the ultrasound image U, displaying the contour line of the blood vessel B, displaying the frame line surrounding the blood vessel B, displaying text, characters, or the like such as an arrow in the vicinity of the blood vessel B.

Further, in a case where the blood vessel B is highlighted on the display device 7, the notification unit 13 can display the blood vessel B with the same color as the color code assigned to the insertion object that the user is trying to insert into the subject. In this manner, the user can check the color code of the insertion object by checking the ultrasound image U displayed on the display device 7, and thereby can easily check the outer diameter of the insertion object.

The ultrasound diagnostic apparatus 1 comprises a speaker (not illustrated) connected to the notification unit 13, and the notification unit 13 can emit voice indicating the determination result of the insertability determination unit 12 from the speaker. In this manner, since the user can grasp the determination result of the insertability determination unit 12 without looking at the display device 7, for example, it is possible to improve the efficiency of the inspection.

Further, in a case where a plurality of blood vessels B are included in the ultrasound image U, for example, the blood vessel extraction unit 8 can extract all the blood vessels B included in the ultrasound image U, and the insertability determination unit 12 can determine whether the insertion object that the user is trying to insert into the subject can be inserted into all the blood vessels B extracted by the blood vessel extraction unit 8.

In this case, the notification unit 13 can highlight the blood vessel B on the display device 7 such that the blood vessel B into which the insertion object can be inserted and the blood vessel B into which the insertion object cannot be inserted are distinguished from each other. For example, the notification unit 13 can display the blood vessel B into which the insertion object can be inserted and the blood vessel B into which the insertion object cannot be inserted, with different colors from each other on the display device 7. Further, for example, the notification unit 13 can display a message such as "OK" indicating that the insertion object can be inserted or a message such as "NG" indicating that the insertion object cannot be inserted, in the vicinity of all the blood vessels B in the ultrasound image U. In this manner, the user can easily grasp the blood vessel B into which the insertion object can be inserted and the blood vessel B into which the insertion object cannot be inserted, by checking the ultrasound image U displayed on the display device 7.

### Second Embodiment

In the first embodiment, the type specifying unit 11 specifies the type of the insertion object that the user is trying to insert into the subject, according to the user's input operation through the input device 15, but the method of specifying the type of the insertion object is not limited thereto. For example, in a case where the insertion object comprises a type information holder such as a two-dimensional code such as a so-called QR code (registered trademark) and a radio frequency identifier (RFID) tag, the type of the insertion object can be specified by reading type information indicating the type of the insertion object, from the type information holder.

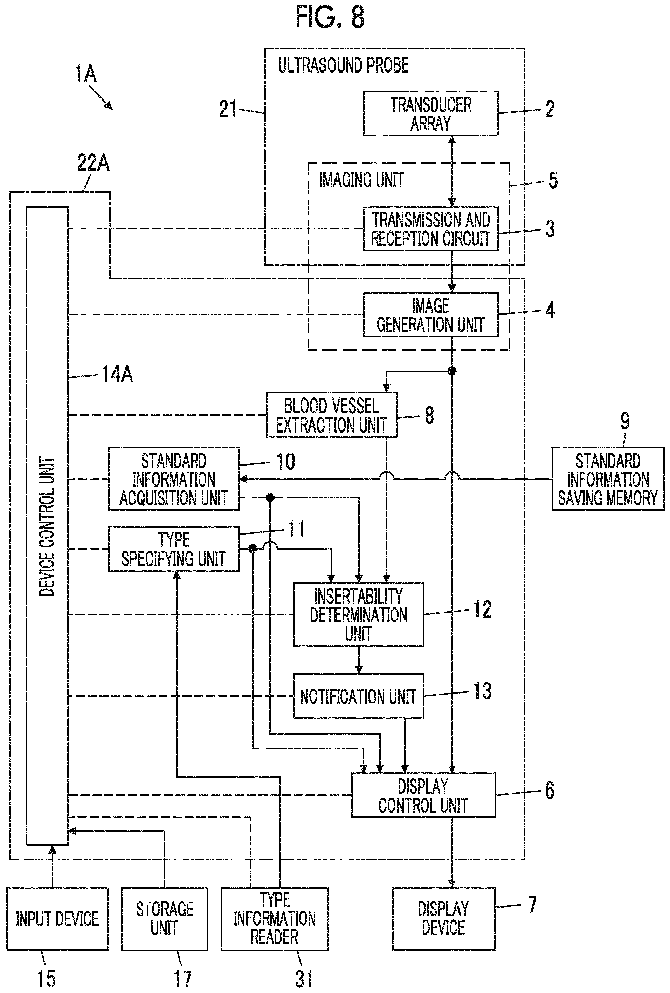

Fig. 8 illustrates a configuration of an ultrasound diagnostic apparatus 1A according to a second embodiment of the present invention. The ultrasound diagnostic apparatus 1A is obtained by excluding the type acceptance unit 16 from the ultrasound diagnostic apparatus 1 of the first embodiment illustrated in Fig. 1, adding a type information reader 31 thereto, comprising a device control unit 14A instead of the device control unit 14, and comprising a processor 22A instead of the processor 22. In the ultrasound diagnostic apparatus 1A, the type information reader 31 is connected to the type specifying unit 11 and the device control unit 14A.

The type information reader 31 reads the type information from the type information holder such as a two-dimensional code and an RFID tag attached to the insertion object. The type information reader 31 sends the read type information to the type specifying unit 11.

The type specifying unit 11 specifies the type of the insertion object that the user is trying to insert into the subject, on the basis of the type information read by the type information reader 31. For example, in a case where it is checked that the vascular insertion object corresponding to the type information read by the type information reader 31 is present in the standard information L acquired by the standard information acquisition unit 10, the type specifying unit 11 can specify the vascular insertion object corresponding to the type information read by the type information reader 31, as the insertion object that the user is trying to insert into the subject.

For example, in a case where it is checked that the vascular insertion object corresponding to the type information read by the type information reader 31 is not present in the standard information L acquired by the standard information acquisition unit 10, the type specifying unit 11 sends the fact to the notification unit 13, and the notification unit 13 can notify of the information indicating that the vascular insertion object corresponding to the type information read by the type information reader 31 is not present in the standard information L.

From the above, with the ultrasound diagnostic apparatus 1A according to the second embodiment, since the type information is read from the type information holder by the type information reader 31 so that the type of the insertion object that the user is trying to insert into the subject is specified, it is possible to more easily specify the type of the insertion object.

The information indicating the type of the insertion object specified by the type specifying unit 11 can be modified by the user's input operation through the input device 15. For example, in a state where the information indicating the type of the insertion object specified by the type specifying unit 11 and the standard information L are displayed on the display device 7, the user selects the vascular insertion object included in the standard information L through the input device 15 so that the information indicating the type of the insertion object specified by the type specifying unit 11 is modified. In this manner, even in a case where the type of the insertion object specified by the type specifying unit 11 is different from the type of the insertion object that the user is trying to insert into the subject, it is possible to easily modify the information indicating the type of the insertion object by the input operation through the input device 15.

### Third Embodiment

For example, an optical image of the insertion object can be captured, and the type of the insertion object that the user is trying to insert into the subject can be specified on the basis of the captured image.

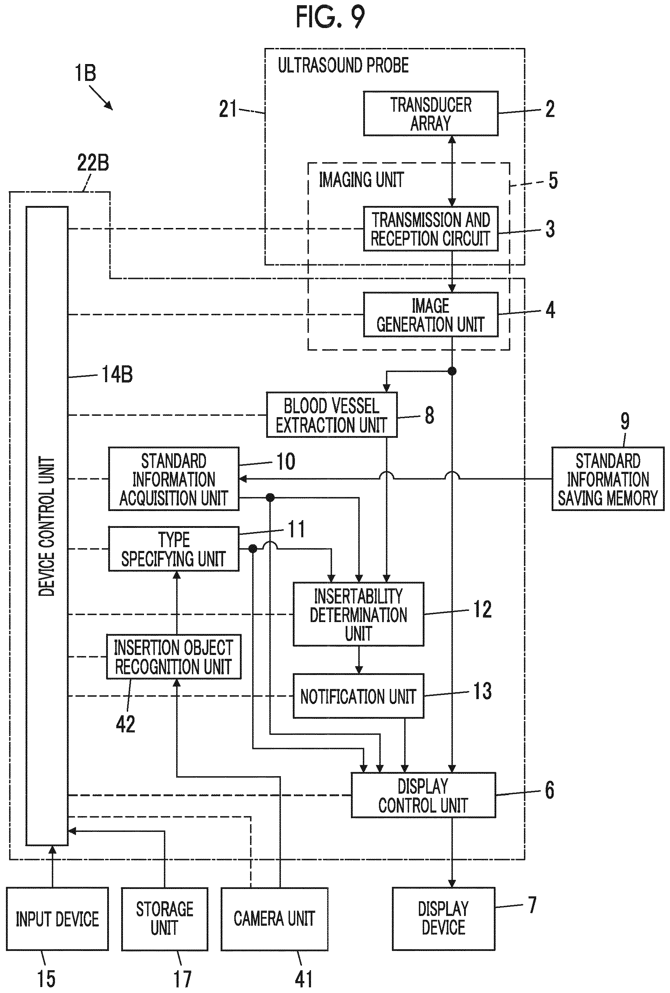

Fig. 9 illustrates a configuration of an ultrasound diagnostic apparatus 1B according to a third embodiment of the present invention. The ultrasound diagnostic apparatus 1B is obtained by excluding the type acceptance unit 16 from the ultrasound diagnostic apparatus 1 of the first embodiment illustrated in Fig. 1, adding a camera unit 41 and an insertion object recognition unit 42 thereto, comprising a device control unit 14B instead of the device control unit 14, and comprising a processor 22B instead of the processor 22. In the ultrasound diagnostic apparatus 1B, the camera unit 41 is connected to the insertion object recognition unit 42, and the type specifying unit 11 and the device control unit 14B are connected to the insertion object recognition unit 42.

The camera unit 41 acquires an optical image of the insertion object by imaging the insertion object. For example, in a case where an instruction to acquire an optical image of the insertion object is input by the user through the input device 15, the device control unit 14B controls the camera unit 41 such that the image of the insertion object is captured, according to the instruction from the user, and thereby the image of the insertion object is captured by the camera unit 41. The camera unit 41 sends the acquired image of the insertion object to the insertion object recognition unit 42.

The insertion object recognition unit 42 can recognize the insertion object on the basis of the image of the insertion object acquired by the camera unit 41, and can automatically discriminate the type of the recognized insertion object. In this case, the insertion object recognition unit 42 can recognize the insertion object and discriminate the type of the recognized insertion object by using, for example, a known algorithm such as template matching, a machine learning method, a general image recognition method using deep learning or the like. The insertion object recognition unit 42 sends the recognition result obtained in this manner to the type specifying unit 11.

The type specifying unit 11 specifies the type of the insertion object on the basis of the insertion object recognized by the insertion object recognition unit 42. In this case, in a case where it is checked that the vascular insertion object corresponding to the insertion object recognized by the insertion object recognition unit 42 is present in the standard information L acquired by the standard information acquisition unit 10, the type specifying unit 11 can specify the vascular insertion object corresponding to the insertion object recognized by the insertion object recognition unit 42, as the insertion object that the user is trying to insert into the subject.

From the above, with the ultrasound diagnostic apparatus 1B according to the third embodiment of the present invention, since the insertion object is recognized by the insertion object recognition unit 42 on the basis of the image captured by the camera unit 41 so that the type of the insertion object that the user is trying to insert into the subject is specified, it is possible to more easily specify the type of the insertion object.

Even in the third embodiment, similarly to the second embodiment, the information indicating the type of the insertion object specified by the type specifying unit 11 can be modified by the user's input operation through the input device 15. In this manner, even in a case where the type of the insertion object specified by the type specifying unit 11 is different from the type of the insertion object that the user is trying to insert into the subject, it is possible to easily modify the information indicating the type of the insertion object by the input operation through the input device 15.

### Fourth Embodiment

For example, the insertion object that is inserted into the subject by the user can be specified by recognizing the insertion object depicted in the ultrasound image U.

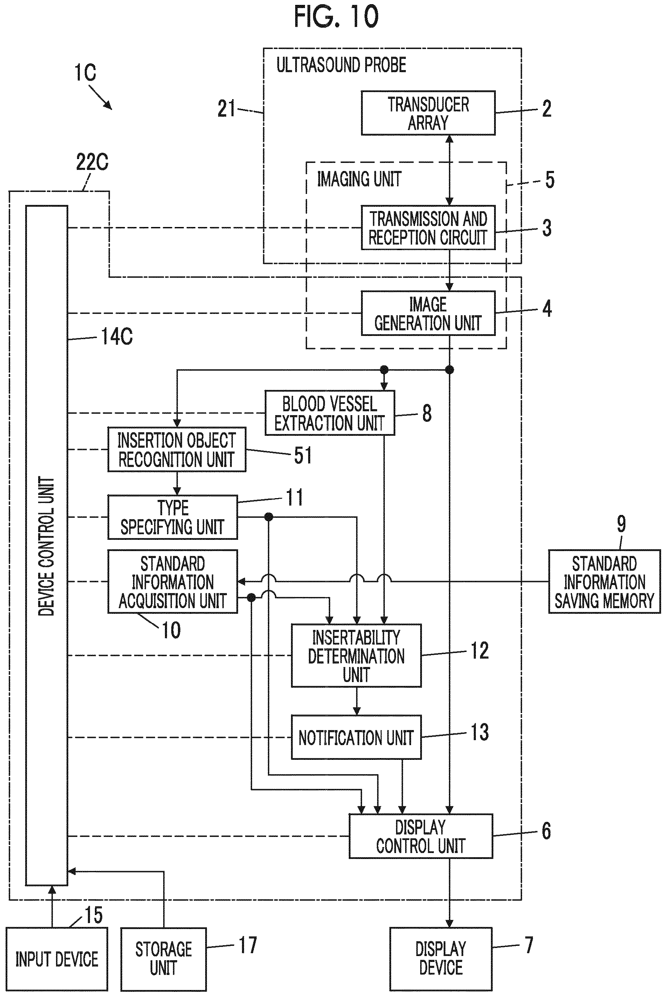

Fig. 10 illustrates a configuration of an ultrasound diagnostic apparatus 1C according to a fourth embodiment of the present invention. The ultrasound diagnostic apparatus 1C is obtained by excluding the type acceptance unit 16 from the ultrasound diagnostic apparatus 1 of the first embodiment illustrated in Fig. 1, adding an insertion object recognition unit 51 thereto, comprising a device control unit 14C instead of the device control unit 14, and comprising a processor 22C instead of the processor 22. In the ultrasound diagnostic apparatus 1C, the insertion object recognition unit 51 is connected to the image generation unit 4, and the type specifying unit 11 and the device control unit 14C are connected to the insertion object recognition unit 51.

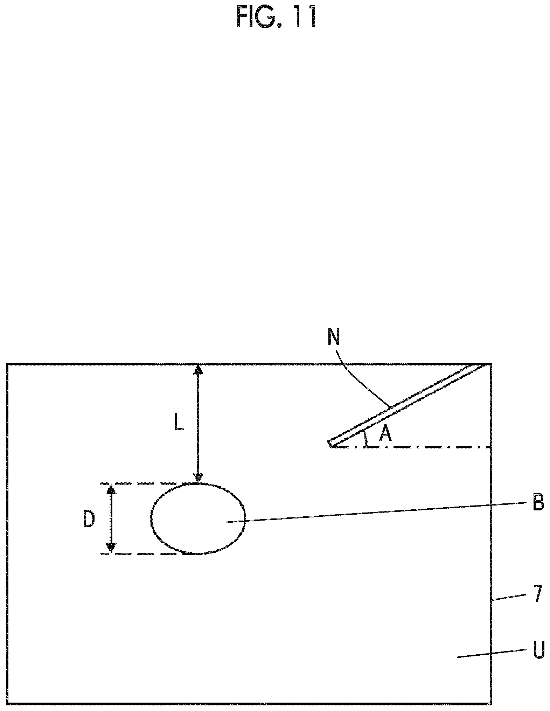

In general, a procedure is known in which the user inserts the insertion object into the subject while bringing the ultrasound probe into contact with the subject and capturing the ultrasound image of the inside of the subject. In a case where such a procedure is performed using the ultrasound diagnostic apparatus 1C, the ultrasound image U including the blood vessel B of the subject and an insertion object N is generated by the image generation unit 4, as illustrated in Fig. 11.

The insertion object recognition unit 51 can recognize the insertion object N on the basis of the ultrasound image U including at least the insertion object N, and can automatically discriminate the type of the recognized insertion object N. For example, in a case where a puncture needle in which a plurality of grooves for reflecting ultrasonic waves are formed, which is a so-called echogenic needle, is used as the insertion object N, the insertion object recognition unit 51 can discriminate the type of the insertion object N by the pattern of the observed grooves. The insertion object recognition unit 51 can recognize the insertion object N included in the ultrasound image U and discriminate the type of the recognized insertion object N by using, for example, a known algorithm such as template matching, a machine learning method, a general image recognition method using deep learning or the like. The insertion object recognition unit 51 sends the recognition result obtained in this manner to the type specifying unit 11.

The type specifying unit 11 specifies the type of the insertion object N on the basis of the insertion object N recognized by the insertion object recognition unit 51. In this case, in a case where it is checked that the vascular insertion object corresponding to the insertion object N recognized by the insertion object recognition unit 51 is present in the standard information L acquired by the standard information acquisition unit 10, the type specifying unit 11 can specify the vascular insertion object corresponding to the insertion object N recognized by the insertion object recognition unit 51, as the insertion object N that is inserted into the subject by the user.

From the above, with the ultrasound diagnostic apparatus 1C according to the fourth embodiment of the present invention, since the insertion object N is recognized by the insertion object recognition unit 51 on the basis of the ultrasound image U in which at least the insertion object N is depicted so that the type of the insertion object N that is inserted into the subject by the user is specified, it is possible to more easily specify the type of the insertion object N.

It has been described that the ultrasound image U is captured in a state where the insertion object N is inserted into the subject, but an ultrasound image U of a so-called phantom, which is a model having almost the same acoustic characteristics as the human body, can be captured in a state where the insertion object N is inserted into the phantom, before the insertion object N is inserted into the subject. In this case, before the insertion object N is inserted into the subject, the type of the insertion object N that the user is trying to insert into the subject can be specified on the basis of the ultrasound image U of the phantom.

### Fifth Embodiment

For example, it can be determined whether the insertion object N that is inserted into the subject by the user can be inserted into the blood vessel B of the subject on the basis of the insertion angle of the insertion object N inserted into the subject.

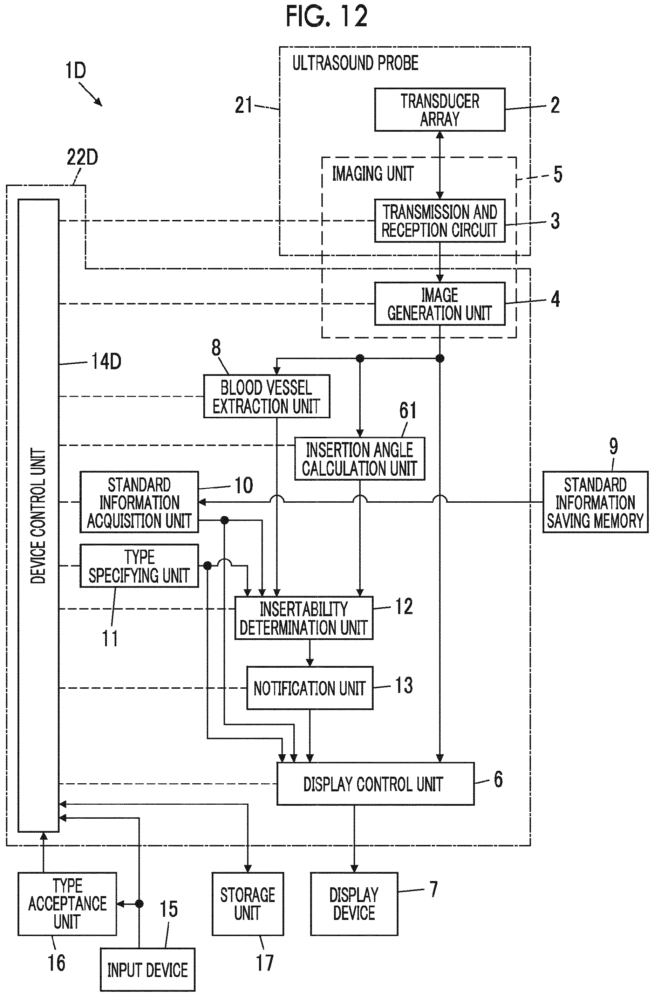

Fig. 12 illustrates a configuration of an ultrasound diagnostic apparatus 1D according to a fifth embodiment of the present invention. The ultrasound diagnostic apparatus 1D is obtained by adding an insertion angle calculation unit 61 to the ultrasound diagnostic apparatus 1 of the first embodiment illustrated in Fig. 1, comprising a device control unit 14D instead of the device control unit 14, and comprising a processor 22D instead of the processor 22. In the ultrasound diagnostic apparatus 1D, the insertion angle calculation unit 61 is connected to the image generation unit 4, and the insertability determination unit 12 and the device control unit 14D are connected to the insertion angle calculation unit 61.

The insertion angle calculation unit 61 recognizes the insertion object N and calculates an insertion angle A thereof, as illustrated in Fig. 11, by analyzing the ultrasound image U including at least the insertion object N. Here, the insertion angle calculation unit 61 can calculate an angle formed by, for example, the recognized insertion object N and a direction orthogonal to the depth direction in the ultrasound image U, as the insertion angle A. The insertion angle calculation unit 61 can recognize the insertion object N included in the ultrasound image U by using, for example, a known algorithm such as template matching, a machine learning method, a general image recognition method using deep learning or the like. The insertion angle calculation unit 61 sends information indicating the calculated insertion angle A to the insertability determination unit 12.

The insertability determination unit 12 can estimate whether or not the insertion object N reaches the blood vessel B of the subject on the assumption that the insertion object N proceeds in the subject while maintaining the insertion angle A calculated by the insertion angle calculation unit 61. In this case, for example, the insertability determination unit 12 acquires the length of the insertion object N specified by the type specifying unit 11 with reference to the standard information L, and estimates whether or not the insertion object N reaches the blood vessel B, on the basis of the distance D from the body surface P of the subject to the blood vessel B, which is measured by the blood vessel extraction unit 8, and the acquired length of the insertion object N. The insertability determination unit 12 can determine that the insertion object N can be inserted into the blood vessel B in a case where it is estimated that the insertion object N reaches the blood vessel B, and can determine that the insertion object N cannot be inserted into the blood vessel B in a case where it is estimated that the insertion object N does not reach the blood vessel B.

From the above, with the ultrasound diagnostic apparatus 1D according to the fifth embodiment of the present invention, since the insertion angle calculation unit 61 calculates the insertion angle A of the insertion object N that is inserted into the subject, and the insertability determination unit 12 determines whether the insertion object N that is inserted into the subject can be inserted into the blood vessel B of the subject, using the insertion angle A of the insertion object N calculated by the insertion angle calculation unit 61, it is possible to improve the accuracy of the determination as compared with a case where the determination is performed on the basis of the recommended insertion angle.

In a case where the insertability determination unit 12 determines that the insertion object N that is inserted into the subject cannot be inserted into the blood vessel B of the subject, the insertion angle A can be calculated so that the insertion object N can be inserted into the blood vessel B of the subject in a case where the insertion object N is inserted into the subject again, for example. Even in this case, the notification unit 13 can notify the insertion angle A calculated by the insertability determination unit 12, to the user.

For example, in a case where a plurality of blood vessels B are depicted in the ultrasound image U generated by the image generation unit 4, the insertability determination unit 12 can determine the insertability of the insertion object N with respect to each of all the blood vessels B included in the ultrasound image U, on the basis of the insertion angle A calculated by the insertion angle calculation unit 61. In this case, the notification unit 13 can highlight, on the display device 7, the blood vessel B, which is determined by the insertability determination unit 12 that the insertion object N can be inserted, among all the blood vessels B included in the ultrasound image U.

Further, it is described that the form of the fifth embodiment is applied to the first embodiment, but the form of the fifth embodiment can be similarly applied to the second embodiment to the fourth embodiment.

### Sixth Embodiment

In the first embodiment, the standard information acquisition unit 10 acquires one piece of standard information L among the plurality of pieces of standard information L saved in advance in the standard information saving memory 9, but can acquire the standard information L via a network, for example.

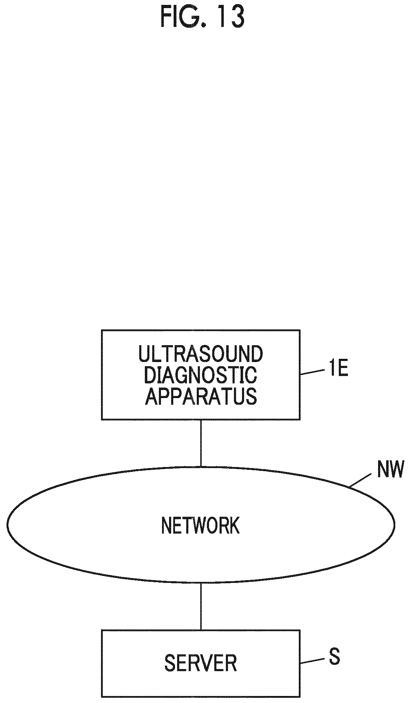

Fig. 13 illustrates an ultrasound diagnostic apparatus 1E according to a sixth embodiment, which is connected to an external server S via a network NW. The ultrasound diagnostic apparatus 1E has the same configuration as the ultrasound diagnostic apparatus 1 according to the first embodiment illustrated in Fig. 1.

The server S saves a plurality of pieces of standard information L corresponding to locations such as various countries, hospitals, and medical departments. The ultrasound diagnostic apparatus 1E can acquire the standard information L saved in the server S.

For example, in a case where positional information indicating the location such as the country, hospital, and medical department where the user performs the ultrasound diagnosis of the subject is input by the user through the input device 15, the standard information L corresponding to the positional information input by the user is downloaded from the server S to the standard information saving memory 9, and the downloaded standard information L is displayed on the display device 7. The standard information acquisition unit 10 acquires the standard information L downloaded from the server S, on the basis of the user's input operation through the input device 15, for example. In this manner, the standard information acquisition unit 10 can acquire the standard information L via the server S.

From the above, with the ultrasound diagnostic apparatus 1E according to the sixth embodiment of the present invention, the standard information L is downloaded from the external server S connected to the ultrasound diagnostic apparatus 1E via the network NW, and the standard information acquisition unit 10 acquires the standard information L downloaded from the server S. Therefore, on the basis of the latest standard information L corresponding to the location such as the country, hospital, and medical department where the ultrasound diagnostic apparatus 1E is positioned, the insertion object N that the user is trying to insert into the subject can be specified and the insertability of the insertion object N that the user is trying to insert into the subject with respect to the blood vessel B of the subject can be determined. In this manner, the insertability of the insertion object N that the user is trying to insert into the subject with respect to the blood vessel B of the subject can be more accurately determined.

Further, with the ultrasound diagnostic apparatus 1E according to the sixth embodiment of the present invention, there is no need to save a huge number of pieces of standard information L corresponding to the location such as the country, hospital, and medical department, in the standard information saving memory 9, and thus it is possible to reduce the amount of information saved in the standard information saving memory 9.

It has been described that the standard information L is downloaded from the server S via the network NW with the user's input operation through the input device 15 as a trigger, but the standard information L can be downloaded on the basis of the positional information measured by a so-called global positioning system (GPS) sensor, for example.

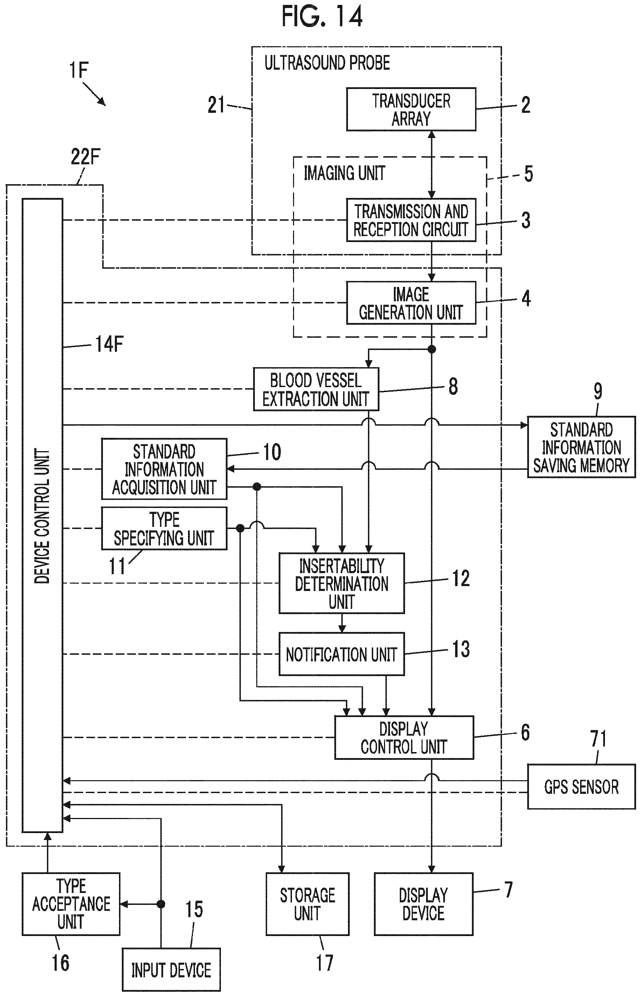

As illustrated in Fig. 14, an ultrasound diagnostic apparatus 1F according to a modification example of the sixth embodiment is obtained by adding a GPS sensor 71 to the ultrasound diagnostic apparatus 1 of the first embodiment illustrated in Fig. 1, comprising a device control unit 14F instead of the device control unit 14, and comprising a processor 22F instead of the processor 22. In the ultrasound diagnostic apparatus 1F, the GPS sensor 71 is connected to the device control unit 14F.

The GPS sensor 71 measures positional information indicating the location such as the country, hospital, and medical department where the ultrasound diagnostic apparatus 1F is positioned. The positional information measured by the GPS sensor 71 is input to the device control unit 14F.

Although not illustrated, the device control unit 14F is connected to the server S via the network NW, and the standard information L is downloaded from the server S on the basis of the positional information of the ultrasound diagnostic apparatus 1F, which is measured by the GPS sensor 71. The standard information L downloaded from the server S is displayed on the display device 7. The standard information acquisition unit 10 acquires the standard information L which is downloaded from the server S and is displayed on the display device 7, on the basis of the user's input operation through the input device 15, for example.

In this manner, the standard information acquisition unit 10 acquires the standard information L corresponding to the positional information measured by the GPS sensor 71, and therefore, the latest standard information L corresponding to the location where the ultrasound diagnostic apparatus 1F is positioned can be easily acquired.

In a case where the standard information saving memory 9 saves the plurality of pieces of standard information L corresponding to locations such as a plurality of countries, hospitals, and medical departments in advance, the standard information L corresponding to the positional information of the ultrasound diagnostic apparatus 1F, which is measured by the GPS sensor 71 can be automatically selected from the plurality of pieces of standard information L saved in the standard information saving memory 9. The standard information acquisition unit 10 acquires the standard information L on the basis of the standard information L that is automatically selected from the plurality of pieces of standard information L saved in the standard information saving memory 9.

Even in such a case, with the ultrasound diagnostic apparatus 1F according to the modification example of the sixth embodiment, the standard information L corresponding to the location where the ultrasound diagnostic apparatus 1F is positioned can be easily acquired.

Further, it is described that the form of the sixth embodiment is applied to the first embodiment, but the form of the sixth embodiment can be similarly applied to the second embodiment to the fifth embodiment.

Although not illustrated, the ultrasound diagnostic apparatuses 1 to 1F of the first embodiment to the sixth embodiment can be configured as a diagnostic apparatus main body having the ultrasound probe 21 and the processors 22 to 22F connected to the ultrasound probe 21. In this case, the diagnostic apparatus main body can be configured by a portable thin computer called a so-called tablet or smartphone. In this manner, the user can easily carry the ultrasound diagnostic apparatuses 1 to 1F and easily perform the ultrasound diagnosis of the subject, and therefore, it is possible to improve the convenience in the ultrasound diagnosis.

### Explanation of References

1, 1A, 1B, 1C, 1D, 1E, 1F: ultrasound diagnostic apparatus
2: transducer array
3: transmission and reception circuit
4: image generation unit
5: imaging unit
6: display control unit
7: display device
8: blood vessel extraction unit
9: standard information saving memory
10: standard information acquisition unit
11: type specifying unit
12: insertability determination unit
13: notification unit
14, 14A, 14B, 14C, 14D, 14F: device control unit
15: input device
16: type acceptance unit
17: storage unit
21: ultrasound probe
22, 22A, 22B, 22C, 22D, 22F: processor
23: pulser
24: amplification unit
25: AD conversion unit
26: beam former
27: signal processing unit
28: DSC
29: image processing unit
31: type information reader
41: camera unit
42, 51: insertion object recognition unit
61: insertion angle calculation unit
71: GPS sensor
A: insertion angle
B: blood vessel
D: distance
G: gel layer
H: diameter
L: standard information
P: body surface
NW: network
S: server
U: ultrasound image

## Claims

1. An ultrasound diagnostic apparatus that displays a blood vessel of a subject into which an insertion object is to be inserted, on an ultrasound image, the ultrasound diagnostic apparatus comprising:
a transducer array (2);
an imaging unit (5) that causes the transducer array (2) to transmit an ultrasound beam toward the subject, and receives an ultrasound echo from the subject to acquire the ultrasound image;
a display device (7) that displays the ultrasound image acquired by the imaging unit (5);
a standard information acquisition unit (10) that acquires standard information including standards relating to at least one type of vascular insertion object;
a type specifying unit (11) that specifies a type of the insertion object that a user is trying to insert into the blood vessel of the subject;
a blood vessel extraction unit (8) that extracts the blood vessel by analyzing the ultrasound image acquired by the imaging unit (5);
an insertability determination unit (12) that determines insertability of the insertion object with respect to the blood vessel extracted by the blood vessel extraction unit (8), on the basis of the standard information acquired by the standard information acquisition unit (10) and the type of the insertion object specified by the type specifying unit (11); and
a notification unit (13) that notifies of a determination result by the insertability determination unit (12);
wherein the insertability determination unit (12) is configured to determine that the insertion object that the user is trying to insert into the subject cannot be inserted into the blood vessel (B) of the subject, in a case where the length of the insertion object specified by the type specifying unit (11) is acquired with reference to the standard information (L) and the acquired length of the insertion object is equal to or less than the distance (D) from the body surface of the subject to the blood vessel (B), which is measured by the blood vessel extraction unit (8);
wherein the insertability determination unit (12) is configured to determine that the insertion object that the user is trying to insert into the subject can be inserted into the blood vessel (B) of the subject, in a case where the length of the insertion object specified by the type specifying unit (11) is acquired with reference to the standard information (L) and the acquired length of the insertion object is longer than the distance (D) from the body surface of the subject to the blood vessel (B), which is measured by the blood vessel extraction unit (8);
wherein the insertability determination unit (12) is configured to determine that the insertion object that the user is trying to insert into the subject cannot be inserted into the blood vessel (B) of the subject, in a case where the length of the insertion object specified by the type specifying unit (11) and a recommended insertion angle of the insertion object are acquired with reference to the standard information (L) and it is estimated that the insertion object cannot reach the blood vessel (B) at the distance (D) from the body surface of the subject, which is measured by the blood vessel extraction unit (8);
wherein the insertability determination unit (12) is configured to determine that the insertion object that the user is trying to insert into the subject can be inserted into the blood vessel (B) of the subject, in a case where the length of the insertion object specified by the type specifying unit (11) and a recommended insertion angle of the insertion object are acquired with reference to the standard information (L) and it is estimated that the insertion object can reach the blood vessel (B) at the distance (D) from the body surface of the subject, which is measured by the blood vessel extraction unit (8);
wherein the insertability determination unit (12) is configured to determine that the insertion object that the user is trying to insert into the subject cannot be inserted into the blood vessel (B), in a case where the outer diameter of the insertion object specified by the type specifying unit is acquired with reference to the standard information (L) and a ratio of the outer diameter of the insertion object to the diameter (H) of the blood vessel (B) measured by the blood vessel extraction unit (8) is greater than a threshold value, even if it is estimated that the insertion object can reach the blood vessel (B); and
wherein the insertability determination unit (12) is configured to determine that the insertion object that the user is trying to insert into the subject can be inserted into the blood vessel (B), in a case where the outer diameter of the insertion object specified by the type specifying unit is acquired with reference to the standard information (L) and a ratio of the outer diameter of the insertion object to the diameter (H) of the blood vessel (B) measured by the blood vessel extraction unit (8) is equal to or less than the threshold value, and
it is estimated that the insertion object can reach the blood vessel (B).

2. The ultrasound diagnostic apparatus according to claim 1, further comprising:
an input device for the user to perform an input operation; and
a type acceptance unit (16) that accepts one type of vascular insertion object selected by the user through the input device on the basis of the at least one type of the vascular insertion object included in the standard information acquired by the standard information acquisition unit (10),
wherein the type specifying unit (11) specifies the type of the insertion object on the basis of the one type of the vascular insertion object accepted by the type acceptance unit (16).

3. The ultrasound diagnostic apparatus according to claim 2,
wherein the standard information acquired by the standard information acquisition unit (10) and the type of the insertion object specified by the type specifying unit (11) are displayed on the display device (7).

4. The ultrasound diagnostic apparatus according to claim 3,
wherein the standard information acquired by the standard information acquisition unit (10) and the type of the insertion object specified by the type specifying unit (11) can be modified by the user through the input device (15).

5. The ultrasound diagnostic apparatus according to claim 1, further comprising:
a type information reader (31) that reads type information from a type information holder attached to the insertion object,
wherein the type specifying unit (11) specifies the type of the insertion object on the basis of the type information read by the type information reader (31).

6. The ultrasound diagnostic apparatus according to claim 1, further comprising:
a camera unit (41) that acquires an image of the insertion object by imaging the insertion object; and
an insertion object recognition unit that recognizes the insertion object on the basis of the image of the insertion object acquired by the camera unit (41),
wherein the type specifying unit (11) specifies the type of the insertion object on the basis of the insertion object recognized by the insertion object recognition unit.

7. The ultrasound diagnostic apparatus according to claim 1, further comprising:
an insertion object recognition unit that recognizes the insertion object on the basis of the ultrasound image acquired by the imaging unit (5),
wherein the type specifying unit (11) specifies the type of the insertion object on the basis of the insertion object recognized by the insertion object recognition unit.

8. The ultrasound diagnostic apparatus according to any one of claims 5 to 7,
wherein the standard information acquired by the standard information acquisition unit (10) and the type of the insertion object specified by the type specifying unit (11) are displayed on the display device (7).

9. The ultrasound diagnostic apparatus according to claim 8, further comprising:
an input device for the user to perform an input operation,
wherein the standard information acquired by the standard information acquisition unit (10) and the type of the insertion object specified by the type specifying unit (11) can be modified by the user through the input device.

10. The ultrasound diagnostic apparatus according to any one of claims 1 to 9,
wherein the notification unit (13) highlights the blood vessel determined by the insertability determination unit (12) that the insertion object can be inserted, on the display device (7).

11. The ultrasound diagnostic apparatus according to claim 10,
wherein the insertion object has a color code assigned according to the standard information, and
the notification unit (13) highlights the blood vessel with the same color as the color code of the insertion object.

12. The ultrasound diagnostic apparatus according to any one of claims 1 to 11,
wherein the ultrasound image includes a plurality of blood vessels extracted by the blood vessel extraction unit (8),
the insertability determination unit (12) determines the insertability of the insertion object with respect to each of the plurality of blood vessels, and
the notification unit (13) displays, among the plurality of blood vessels, the blood vessel determined by the insertability determination unit (12) that the insertion object can be inserted and the blood vessel determined by the insertability determination unit (12) that the insertion object cannot be inserted, on the display device (7) in a distinguishable manner.

13. The ultrasound diagnostic apparatus according to claim 12, further comprising:
an insertion angle calculation unit that calculates an insertion angle of the insertion object by analyzing the ultrasound image acquired by the imaging unit (5),
wherein the insertability determination unit (12) determines the insertability of the insertion object with respect to each of the plurality of blood vessels on the basis of the insertion angle calculated by the insertion angle calculation unit, and
the notification unit (13) highlights, among the plurality of blood vessels, the blood vessel determined by the insertability determination unit (12) that the insertion object can be inserted, on the display device (7).

14. The ultrasound diagnostic apparatus according to any one of claims 1 to 13, further comprising:
a standard information saving memory (9) that saves a plurality of pieces of the standard information,
wherein the standard information acquisition unit (10) acquires one piece of the standard information among the plurality of pieces of standard information saved in the standard information saving memory (9).

15. The ultrasound diagnostic apparatus according to any one of claims 1 to 13,
wherein the ultrasound diagnostic apparatus is connected to an external server via a network, and
the standard information acquisition unit (10) acquires the standard information via the server.

16. The ultrasound diagnostic apparatus according to claim 15, further comprising:
a GPS sensor that measures positional information of the ultrasound diagnostic apparatus,
wherein the standard information acquisition unit (10) acquires the standard information corresponding to the positional information measured by the GPS sensor.

17. A control method of an ultrasound diagnostic apparatus that displays a blood vessel of a subject into which an insertion object is to be inserted, on an ultrasound image, the control method comprising:
causing a transducer array (2) to transmit an ultrasound beam toward the subject, and receiving an ultrasound echo from the subject to acquire the ultrasound image;
displaying the acquired ultrasound image;
acquiring standard information including standards relating to at least one type of vascular insertion object;
specifying a type of the insertion object that a user is trying to insert into the blood vessel of the subject;
extracting the blood vessel by analyzing the acquired ultrasound image;
determining insertability of the insertion object with respect to the extracted blood vessel on the basis of the acquired standard information and the specified type of the insertion object; and
notifying of a determination result;
wherein the step of determining insertability of the insertion object comprises:
determining that the insertion object that the user is trying to insert into the subject cannot be inserted into the blood vessel (B) of the subject in a case where the length of the insertion object specified by the type specifying unit (11) is acquired with reference to the standard information (L) and the acquired length of the insertion object is equal to or less than the distance (D) from the body surface of the subject to the blood vessel (B), which is measured by the blood vessel extraction unit (8);
determining that the insertion object that the user is trying to insert into the subject can be inserted into the blood vessel (B) of the subject, in a case where the length of the insertion object specified by the type specifying unit (11) is acquired with reference to the standard information (L) and the acquired length of the insertion object is longer than the distance (D) from the body surface of the subject to the blood vessel (B), which is measured by the blood vessel extraction unit (8);
determining that the insertion object that the user is trying to insert into the subject cannot be inserted into the blood vessel (B) of the subject, in a case where the length of the insertion object specified by the type specifying unit (11) and a recommended insertion angle of the insertion object are acquired with reference to the standard information (L) and it is estimated that the insertion object cannot reach the blood vessel (B) at the distance (D) from the body surface of the subject, which is measured by the blood vessel extraction unit (8);
determining that the insertion object that the user is trying to insert into the subject can be inserted into the blood vessel (B) of the subject, in a case where the length of the insertion object specified by the type specifying unit (11) and a recommended insertion angle of the insertion object are acquired with reference to the standard information (L) and it is estimated that the insertion object can reach the blood vessel (B) at the distance (D) from the body surface of the subject, which is measured by the blood vessel extraction unit (8);
determining that the insertion object that the user is trying to insert into the subject cannot be inserted into the blood vessel (B), in a case where the outer diameter of the insertion object specified by the type specifying unit is acquired with reference to the standard information (L) and a ratio of the outer diameter of the insertion object specified by the type specifying unit (11) to the diameter (H) of the blood vessel (B) measured by the blood vessel extraction unit (8) is greater than a threshold value, even if it is estimated that the insertion object can reach the blood vessel (B); and
determining that the insertion object that the user is trying to insert into the subject can be inserted into the blood vessel (B), in a case where the outer diameter of the insertion object specified by the type specifying unit is acquired with reference to the standard information (L) and a ratio of the outer diameter of the insertion object specified by the type specifying unit (11) to the diameter (H) of the blood vessel (B) measured by the blood vessel extraction unit (8) is equal to or less than the threshold value and it is estimated that the insertion object can reach the blood vessel (B).

## Patentansprüche

1. Ultraschalldiagnosevorrichtung, die ein Blutgefäß einer Untersuchungsperson, in das ein Einführungsobjekt einzuführen ist, auf einem Ultraschallbild anzeigt, wobei die Ultraschalldiagnosevorrichtung umfasst:
eine Wandleranordnung (2);
eine Bildgebungseinheit (5), die die Wandleranordnung (2) veranlasst, einen Ultraschallstrahl zu der Untersuchungsperson hin zu transmittieren, und ein Ultraschallecho von der Untersuchungsperson empfängt, um das Ultraschallbild zu erfassen;
eine Anzeigevorrichtung (7), die das von der Bildgebungseinheit (5) erfasste Ultraschallbild anzeigt;
eine Standardinformations-Erfassungseinheit (10), die Standardinformationen einschließlich Standards in Bezug auf mindestens einen Typ von vaskulärem Einführungsobjekt erfasst;
eine Typspezifizierungseinheit (11), die einen Typ des Einführungsobjekts spezifiziert, das ein Benutzer in das Blutgefäß der Untersuchungsperson einzuführen versucht;
eine Blutgefäß-Extraktionseinheit (8), die das Blutgefäß durch Analysieren des von der Bildgebungseinheit (5) erfassten Ultraschallbildes extrahiert;
eine Einführbarkeits-Bestimmungseinheit (12), die Einführbarkeit des Einführungsobjekts in Bezug auf das von der Blutgefäß-Extraktionseinheit (8) extrahierte Blutgefäß auf der Grundlage der von der Standardinformations-Erfassungseinheit (10) erfassten Standardinformationen und des von der Typspezifizierungseinheit (11) spezifizierten Typs des Einführungsobjekts bestimmt; und
eine Benachrichtigungseinheit (13), die über ein Bestimmungsergebnis durch die Einführbarkeits-Bestimmungseinheit (12) benachrichtigt;
wobei die Einführbarkeits-Bestimmungseinheit (12) so konfiguriert ist, dass sie in einem Fall, in dem die Länge des Einführungsobjekts, die von der Typspezifizierungseinheit (11) spezifiziert wird, unter Bezugnahme auf die Standardinformationen (L) erfasst wird und die erfasste Länge des Einführungsobjekts gleich oder kleiner als der Abstand (D) von der Körperoberfläche der Untersuchungsperson zu dem Blutgefäß (B) ist, der von der Blutgefäß-Extraktionseinheit (8) gemessen wird, bestimmt, dass das Einführungsobjekt, das der Benutzer in die Untersuchungsperson einzuführen versucht, nicht in das Blutgefäß (B) der Untersuchungsperson eingeführt werden kann;
wobei die Einführbarkeits-Bestimmungseinheit (12) so konfiguriert ist, dass sie in einem Fall, in dem die Länge des Einführungsobjekts, die von der Typspezifizierungseinheit (11) spezifiziert wird, unter Bezugnahme auf die Standardinformationen (L) erfasst wird und die erfasste Länge des Einführungsobjekts länger als der Abstand (D) von der Körperoberfläche der Untersuchungsperson zu dem Blutgefäß (B) ist, der von der Blutgefäß-Extraktionseinheit (8) gemessen wird, bestimmt, dass das Einführungsobjekt, das der Benutzer in die Untersuchungsperson einzuführen versucht, in das Blutgefäß (B) der Untersuchungsperson eingeführt werden kann;
wobei die Einführbarkeits-Bestimmungseinheit (12) so konfiguriert ist, dass sie in einem Fall, in dem die Länge des Einführungsobjekts, die von der Typspezifizierungseinheit (11) spezifiziert wird, und ein empfohlener Einführwinkel des Einführungsobjekts unter Bezugnahme auf die Standardinformationen (L) erfasst werden und es geschätzt wird, dass das Einführungsobjekt das Blutgefäß (B) an dem Abstand (D) von der Körperoberfläche der Untersuchungsperson, der von der Blutgefäß-Extraktionseinheit (8) gemessen wird, nicht erreichen kann, bestimmt, dass das Einführungsobjekt, das der Benutzer in die Untersuchungsperson einzuführen versucht, nicht in das Blutgefäß (B) der Untersuchungsperson eingeführt werden kann;
wobei die Einführbarkeits-Bestimmungseinheit (12) so konfiguriert ist, dass sie in einem Fall, in dem die Länge des Einführungsobjekts, die von der Typspezifizierungseinheit (11) spezifiziert wird, und ein empfohlener Einführwinkel des Einführungsobjekts unter Bezugnahme auf die Standardinformationen (L) erfasst werden und es geschätzt wird, dass das Einführungsobjekt das Blutgefäß (B) an dem Abstand (D) von der Körperoberfläche der Untersuchungsperson, der von der Blutgefäß-Extraktionseinheit (8) gemessen wird, erreichen kann, bestimmt, dass das Einführungsobjekt, das der Benutzer in die Untersuchungsperson einzuführen versucht, in das Blutgefäß (B) der Untersuchungsperson eingeführt werden kann;
wobei die Einführbarkeits-Bestimmungseinheit (12) so konfiguriert ist, dass sie in einem Fall, in dem der Außendurchmesser des Einführungsobjekts, der von der Typspezifizierungseinheit spezifiziert wird, unter Bezugnahme auf die Standardinformationen (L) erfasst wird und ein Verhältnis des Außendurchmessers des Einführungsobjekts zu dem Durchmesser (H) des Blutgefäßes (B), der von der Blutgefäß-Extraktionseinheit (8) gemessen wird, größer als ein Schwellenwert ist, bestimmt, dass das Einführungsobjekt, das der Benutzer in die Untersuchungsperson einzuführen versucht, in das Blutgefäß (B) nicht eingeführt werden kann, selbst wenn geschätzt wird, dass das Einführungsobjekt das Blutgefäß (B) erreichen kann; und
wobei die Einführbarkeits-Bestimmungseinheit (12) so konfiguriert ist, dass sie in einem Fall, in dem der Außendurchmesser des Einführungsobjekts, der von der Typspezifizierungseinheit spezifiziert wird, unter Bezugnahme auf die Standardinformationen (L) erfasst wird und ein Verhältnis des Außendurchmessers des Einführungsobjekts zu dem Durchmesser (H) des Blutgefäßes (B), der von der Blutgefäß-Extraktionseinheit (8) gemessen wird, gleich oder kleiner als der Schwellenwert ist und es geschätzt wird, dass das Einführungsobjekt das Blutgefäß (B) erreichen kann, bestimmt, dass das Einführungsobjekt, das der Benutzer in die Untersuchungsperson einzuführen versucht, in das Blutgefäß (B) eingeführt werden kann.

2. Ultraschalldiagnosevorrichtung nach Anspruch 1, ferner umfassend:
eine Eingabevorrichtung für den Benutzer, um eine Eingabebedienung durchzuführen; und
eine Typannahmeeinheit (16), die einen Typ von vaskulärem Einführungsobjekt, der von dem Benutzer durch die Eingabevorrichtung ausgewählt wird, auf der Grundlage des mindestens einen Typs des vaskulären Einführungsobjekts, der in den von der Standardinformations-Erfassungseinheit (10) erfassten Standardinformationen enthalten ist, annimmt,
wobei die Typspezifizierungseinheit (11) den Typ des Einführungsobjekts auf der Grundlage des einen Typs des vaskulären Einführungsobjekts, der von der Typannahmeeinheit (16) angenommen wird, spezifiziert.

3. Ultraschalldiagnosevorrichtung nach Anspruch 2,
wobei die von der Standardinformations-Erfassungseinheit (10) erfassten Standardinformationen und der von der Typspezifizierungseinheit (11) spezifizierte Typ des Einführungsobjekts auf der Anzeigevorrichtung (7) angezeigt werden.

4. Ultraschalldiagnosevorrichtung nach Anspruch 3,
wobei die von der Standardinformations-Erfassungseinheit (10) erfassten Standardinformationen und der von der Typspezifizierungseinheit (11) spezifizierte Typ des Einführungsobjekts von dem Benutzer über die Eingabevorrichtung (15) modifiziert werden können.

5. Ultraschalldiagnosevorrichtung nach Anspruch 1, ferner umfassend:
einen Typinformationsleser (31), der Typinformationen aus einem Typinformationsspeicher, der an dem Einführungsobjekt angebracht ist, liest,
wobei die Typspezifizierungseinheit (11) den Typ des Einführungsobjekts auf der Grundlage der von dem Typinformationsleser (31) gelesenen Typinformationen spezifiziert.

6. Ultraschalldiagnosevorrichtung nach Anspruch 1, ferner umfassend:
eine Kameraeinheit (41), die ein Bild des Einführungsobjekts durch Abbilden des Einführungsobjekts erfasst; und
eine Einführungsobjekt-Erkennungseinheit, die das Einführungsobjekt auf der Grundlage des von der Kameraeinheit (41) erfassten Bildes des Einführungsobjekts erkennt,
wobei die Typspezifizierungseinheit (11) den Typ des Einführungsobjekts auf der Grundlage des von der Einführungsobjekt-Erkennungseinheit erkannten Einführungsobjekts spezifiziert.

7. Ultraschalldiagnosevorrichtung nach Anspruch 1, ferner umfassend:
eine Einführungsobjekt-Erkennungseinheit, die das Einführungsobjekt auf der Grundlage des von der Bildgebungseinheit (5) erfassten Ultraschallbildes erkennt,
wobei die Typspezifizierungseinheit (11) den Typ des Einführungsobjekts auf der Grundlage des von der Einführungsobjekt-Erkennungseinheit erkannten Einführungsobjekts spezifiziert.

8. Ultraschalldiagnosevorrichtung nach einem der Ansprüche 5 bis 7,
wobei die von der Standardinformations-Erfassungseinheit (10) erfassten Standardinformationen und der von der Typspezifizierungseinheit (11) spezifizierte Typ des Einführungsobjekts auf der Anzeigevorrichtung (7) angezeigt werden.

9. Ultraschalldiagnosevorrichtung nach Anspruch 8, ferner umfassend:
eine Eingabevorrichtung für den Benutzer, um eine Eingabebedienung durchzuführen,
wobei die von der Standardinformations-Erfassungseinheit (10) erfassten Standardinformationen und der von der Typspezifizierungseinheit (11) spezifizierte Typ des Einführungsobjekts von dem Benutzer über die Eingabevorrichtung modifiziert werden können.

10. Ultraschalldiagnosevorrichtung nach einem der Ansprüche 1 bis 9,
wobei die Benachrichtigungseinheit (13) das Blutgefäß, das durch die Einführbarkeits-Bestimmungseinheit (12) bestimmt worden ist, dass das Einführungsobjekt eingeführt werden kann, auf der Anzeigevorrichtung (7) hervorhebt.

11. Ultraschalldiagnosevorrichtung nach Anspruch 10,
wobei das Einführungsobjekt einen Farbcode aufweist, der gemäß den Standardinformationen zugeordnet ist, und
die Benachrichtigungseinheit (13) das Blutgefäß mit derselben Farbe wie der Farbcode des Einführungsobjekts hervorhebt.

12. Ultraschalldiagnosevorrichtung nach einem der Ansprüche 1 bis 11,
wobei das Ultraschallbild mehrere Blutgefäße enthält, die von der Blutgefäß-Extraktionseinheit (8) extrahiert worden sind,
die Einführbarkeits-Bestimmungseinheit (12) die Einführbarkeit des Einführungsobjekts in Bezug auf jedes der mehreren Blutgefäße bestimmt, und die Benachrichtigungseinheit (13) unter den mehreren Blutgefäßen das Blutgefäß, das durch die Einführbarkeits-Bestimmungseinheit (12) bestimmt worden ist, dass das Einführungsobjekt eingeführt werden kann, und das Blutgefäß, das durch die Einführbarkeits-Bestimmungseinheit (12) bestimmt worden ist, dass das Einführungsobjekt nicht eingeführt werden kann, auf der Anzeigevorrichtung (7) auf unterscheidbare Weise anzeigt.

13. Ultraschalldiagnosevorrichtung nach Anspruch 12, ferner umfassend:
eine Einführwinkel-Berechnungseinheit, die einen Einführwinkel des Einführungsobjekts durch Analysieren des von der Bildgebungseinheit (5) erfassten Ultraschallbildes berechnet,
wobei die Einführbarkeits-Bestimmungseinheit (12) die Einführbarkeit des Einführungsobjekts in Bezug auf jedes der mehreren Blutgefäße auf der Grundlage des von der Einführwinkel-Berechnungseinheit berechneten Einführwinkels bestimmt, und
die Benachrichtigungseinheit (13) unter den mehreren Blutgefäßen das Blutgefäß, das durch die Einführbarkeits-Bestimmungseinheit (12) bestimmt worden ist, dass das Einführungsobjekt eingeführt werden kann, auf der Anzeigevorrichtung (7) hervorhebt.

14. Ultraschalldiagnosevorrichtung nach einem der Ansprüche 1 bis 13, ferner umfassend:
einen Standardinformationsspeicher (9), der mehrere der Standardinformationen speichert,
wobei die Standardinformations-Erfassungseinheit (10) eine Angabe der Standardinformationen unter den mehreren Standardinformationen, die in dem Standardinformationsspeicher (9) gespeichert sind, erfasst.

15. Ultraschalldiagnosevorrichtung nach einem der Ansprüche 1 bis 13,
wobei die Ultraschalldiagnosevorrichtung via ein Netzwerk mit einem externen Server verbunden ist, und
die Standardinformations-Erfassungseinheit (10) die Standardinformationen via den Server erfasst.

16. Ultraschalldiagnosevorrichtung nach Anspruch 15, ferner umfassend:
einen GPS-Sensor, der Positionsinformationen der Ultraschalldiagnosevorrichtung misst,
wobei die Standardinformations-Erfassungseinheit (10) die Standardinformationen, die den von dem GPS-Sensor gemessenen Positionsinformationen entsprechen, erfasst.

17. Steuerverfahren einer Ultraschalldiagnosevorrichtung, die ein Blutgefäß einer Untersuchungsperson, in das ein Einführungsobjekt einzuführen ist, auf einem Ultraschallbild anzeigt, wobei das Steuerverfahren umfasst:
Veranlassen einer Wandleranordnung (2), einen Ultraschallstrahl zu der Untersuchungsperson hin zu transmittieren, und Empfangen eines Ultraschalle chos von der Untersuchungsperson, um das Ultraschallbild zu erfassen;
Anzeigen des erfassten Ultraschallbildes;
Erfassen von Standardinformationen einschließlich Standards in Bezug auf mindestens einen Typ von vaskulärem Einführungsobjekt;
Spezifizieren eines Typs des Einführungsobjekts, das ein Benutzer in das Blutgefäß der Untersuchungsperson einzuführen versucht;
Extrahieren des Blutgefäßes durch Analysieren des erfassten Ultraschallbildes;
Bestimmen von Einführbarkeit des Einführungsobjekts in Bezug auf das extrahierte Blutgefäß auf der Grundlage der erfassten Standardinformationen und des spezifizierten Typs des Einführungsobjekts; und
Benachrichtigen über ein Bestimmungsergebnis;
wobei der Schritt des Bestimmens von Einführbarkeit des Einführungsobjekts umfasst:
Bestimmen, dass das Einführungsobjekt, das der Benutzer in die Untersuchungsperson einzuführen versucht, nicht in das Blutgefäß (B) der Untersuchungsperson eingeführt werden kann, in einem Fall, in dem die Länge des Einführungsobjekts, die von der Typspezifizierungseinheit (11) spezifiziert wird, unter Bezugnahme auf die Standardinformationen (L) erfasst wird und die erfasste Länge des Einführungsobjekts gleich oder kleiner als der Abstand (D) von der Körperoberfläche der Untersuchungsperson zu dem Blutgefäß (B) ist, der von der Blutgefäß-Extraktionseinheit (8) gemessen wird;
Bestimmen, dass das Einführungsobjekt, das der Benutzer in die Untersuchungsperson einzuführen versucht, in das Blutgefäß (B) der Untersuchungsperson eingeführt werden kann, in einem Fall, in dem die Länge des Einführungsobjekts, die von der Typspezifizierungseinheit (11) spezifiziert wird, unter Bezugnahme auf die Standardinformationen (L) erfasst wird und die erfasste Länge des Einführungsobjekts länger als der Abstand (D) von der Körperoberfläche der Untersuchungsperson zu dem Blutgefäß (B) ist, der von der Blutgefäß-Extraktionseinheit (8) gemessen wird;
Bestimmen, dass das Einführungsobjekt, das der Benutzer in die Untersuchungsperson einzuführen versucht, nicht in das Blutgefäß (B) der Untersuchungsperson eingeführt werden kann, in einem Fall, in dem die Länge des Einführungsobjekts, die von der Typspezifizierungseinheit (11) spezifiziert wird, und ein empfohlener Einführwinkel des Einführungsobjekts unter Bezugnahme auf die Standardinformationen (L) erfasst werden und es geschätzt wird, dass das Einführungsobjekt das Blutgefäß (B) an dem Abstand (D) von der Körperoberfläche der Untersuchungsperson, der von der Blutgefäß-Extraktionseinheit (8) gemessen wird, nicht erreichen kann;
Bestimmen, dass das Einführungsobjekt, das der Benutzer in die Untersuchungsperson einzuführen versucht, in das Blutgefäß (B) der Untersuchungsperson eingeführt werden kann, in einem Fall, in dem die Länge des Einführungsobjekts, die von der Typspezifizierungseinheit (11) spezifiziert wird, und ein empfohlener Einführwinkel des Einführungsobjekts unter Bezugnahme auf die Standardinformationen (L) erfasst werden und es geschätzt wird, dass das Einführungsobjekt das Blutgefäß (B) an dem Abstand (D) von der Körperoberfläche der Untersuchungsperson, der von der Blutgefäß-Extraktionseinheit (8) gemessen wird, erreichen kann;
Bestimmen, dass das Einführungsobjekt, das der Benutzer in die Untersuchungsperson einzuführen versucht, nicht in das Blutgefäß (B) eingeführt werden kann, in einem Fall, in dem der Außendurchmesser des Einführungsobjekts, der von der Typspezifizierungseinheit spezifiziert wird, unter Bezugnahme auf die Standardinformationen (L) erfasst wird und ein Verhältnis des Außendurchmessers des Einführungsobjekts, der von der Typspezifizierungseinheit (11) spezifiziert wird, zu dem Durchmesser (H) des Blutgefäßes (B), der von der Blutgefäß-Extraktionseinheit (8) gemessen wird, größer als ein Schwellenwert ist, selbst wenn geschätzt wird, dass das Einführungsobjekt das Blutgefäß (B) erreichen kann; und
Bestimmen, dass das Einführungsobjekt, das der Benutzer in die Untersuchungsperson einzuführen versucht, in das Blutgefäß (B) eingeführt werden kann, in einem Fall, in dem der Außendurchmesser des Einführungsobjekts, der von der Typspezifizierungseinheit spezifiziert wird, unter Bezugnahme auf die Standardinformationen (L) erfasst wird und ein Verhältnis des Außendurchmessers des Einführungsobjekts, der von der Typspezifizierungseinheit (11) spezifiziert wird, zu dem Durchmesser (H) des Blutgefäßes (B), der von der Blutgefäß-Extraktionseinheit (8) gemessen wird, gleich oder kleiner als der Schwellenwert ist und es geschätzt wird, dass das Einführungsobjekt das Blutgefäß (B) erreichen kann.

## Revendications

1. Appareil de diagnostic par ultrasons qui affiche un vaisseau sanguin d'un sujet dans lequel un objet d'insertion doit être inséré, sur une image ultrasonore, l'appareil de diagnostic par ultrasons comprenant :
un réseau de transducteurs (2) ;
une unité d'imagerie (5) qui amène le réseau de transducteurs (2) à transmettre un faisceau ultrasonore vers le sujet, et reçoit un écho ultrasonore du sujet pour acquérir l'image ultrasonore ;
un dispositif d'affichage (7) qui affiche l'image ultrasonore acquise par l'unité d'imagerie (5) ;
une unité d'acquisition d'informations standard (10) qui acquiert des informations standard incluant des normes concernant au moins un type d'objet d'insertion vasculaire ;
une unité de spécification de type (11) qui spécifie un type de l'objet d'insertion que l'utilisateur essaie d'insérer dans le vaisseau sanguin du sujet ;
une unité d'extraction de vaisseau sanguin (8) qui extrait le vaisseau sanguin en analysant l'image ultrasonore acquise par l'unité d'imagerie (5) ;
une unité de détermination d'insertabilité (12) qui détermine l'insertabilité de l'objet d'insertion par rapport au vaisseau sanguin extrait par l'unité d'extraction de vaisseau sanguin (8), sur la base des informations standard acquises par l'unité d'acquisition d'informations standard (10) et du type de l'objet d'insertion spécifié par l'unité de spécification de type (11) ; et
une unité de notification (13) qui notifie d'un résultat de détermination par l'unité de détermination d'insertabilité (12) ;
dans lequel l'unité de détermination d'insertabilité (12) est configurée pour déterminer que l'objet d'insertion que l'utilisateur essaie d'insérer dans le sujet ne peut pas être inséré dans le vaisseau sanguin (B) du sujet, dans un cas où la longueur de l'objet d'insertion spécifiée par l'unité de spécification de type (11) est acquise en référence aux informations standard (L) et la longueur acquise de l'objet d'insertion est égale ou inférieure à la distance (D) depuis la surface corporelle du sujet jusqu'au vaisseau sanguin (B), qui est mesurée par l'unité d'extraction de vaisseau sanguin (8) ;
dans lequel l'unité de détermination d'insertabilité (12) est configurée pour déterminer que l'objet d'insertion que l'utilisateur essaie d'insérer dans le sujet peut être inséré dans le vaisseau sanguin (B) du sujet, dans un cas où la longueur de l'objet d'insertion spécifiée par l'unité de spécification de type (11) est acquise en référence aux informations standard (L) et la longueur acquise de l'objet d'insertion est plus longue que la distance (D) depuis la surface corporelle du sujet jusqu'au vaisseau sanguin (B), qui est mesurée par l'unité d'extraction de vaisseau sanguin (8) ;
dans lequel l'unité de détermination d'insertabilité (12) est configurée pour déterminer que l'objet d'insertion que l'utilisateur essaie d'insérer dans le sujet ne peut pas être inséré dans le vaisseau sanguin (B) du sujet, dans un cas où la longueur de l'objet d'insertion spécifiée par l'unité de spécification de type (11) et un angle d'insertion recommandé de l'objet d'insertion sont acquises en référence aux informations standard (L) et il est estimé que l'objet d'insertion ne peut pas atteindre le vaisseau sanguin (B) à la distance (D) depuis la surface corporelle du sujet, qui est mesurée par l'unité d'extraction de vaisseau sanguin (8) ;
dans lequel l'unité de détermination d'insertabilité (12) est configurée pour déterminer que l'objet d'insertion que l'utilisateur essaie d'insérer dans le sujet peut être inséré dans le vaisseau sanguin (B) du sujet, dans un cas où la longueur de l'objet d'insertion spécifiée par l'unité de spécification de type (11) et un angle d'insertion recommandé de l'objet d'insertion sont acquises en référence aux informations standard (L) et il est estimé que l'objet d'insertion peut atteindre le vaisseau sanguin (B) à la distance (D) depuis la surface corporelle du sujet, qui est mesurée par l'unité d'extraction de vaisseau sanguin (8) ;
dans lequel l'unité de détermination d'insertabilité (12) est configurée pour déterminer que l'objet d'insertion que l'utilisateur essaie d'insérer dans le sujet ne peut pas être inséré dans le vaisseau sanguin (B), dans un cas où le diamètre extérieur de l'objet d'insertion spécifié par l'unité de spécification de type est acquis en référence aux informations standard (L) et un rapport du diamètre extérieur de l'objet d'insertion au diamètre (H) du vaisseau sanguin (B) mesuré par l'unité d'extraction de vaisseau sanguin (8) est supérieur à une valeur seuil, même si l'on estime que l'objet d'insertion peut atteindre le vaisseau sanguin (B) ; et
dans lequel l'unité de détermination d'insertabilité (12) est configurée pour déterminer que l'objet d'insertion que l'utilisateur essaie d'insérer dans le sujet peut être inséré dans le vaisseau sanguin (B), dans un cas où le diamètre extérieur de l'objet d'insertion spécifié par l'unité de spécification de type est acquis en référence aux informations standard (L) et un rapport du diamètre extérieur de l'objet d'insertion au diamètre (H) du vaisseau sanguin (B) mesuré par l'unité d'extraction de vaisseau sanguin (8) est égal ou inférieur à la valeur seuil, et il est estimé que l'objet d'insertion peut atteindre le vaisseau sanguin (B).

2. Appareil de diagnostic par ultrasons selon la revendication 1, comprenant en outre :
un dispositif d'entrée permettant á l'utilisateur d'effectuer une opération d'entrée ; et
une unité d'acceptation de type (16) qui accepte un type d'objet d'insertion vasculaire sélectionné par l'utilisateur par le dispositif d'entrée sur la base de l'au moins un type de l'objet d'insertion vasculaire inclus dans les informations standard acquises par l'unité d'acquisition d'informations standard (10),
dans lequel l'unité de spécification de type (11) spécifie le type de l'objet d'insertion sur la base du type d'objet d'insertion vasculaire accepté par l'unité d'acceptation de type (16).

3. Appareil de diagnostic par ultrasons selon la revendication 2,
dans lequel les informations standard acquises par l'unité d'acquisition d'informations standard (10) et le type de l'objet d'insertion spécifié par l'unité de spécification de type (11) sont affichées sur le dispositif d'affichage (7).

4. Appareil de diagnostic par ultrasons selon la revendication 3,
dans lequel les informations standard acquises par l'unité d'acquisition d'informations standard (10) et le type de l'objet d'insertion spécifié par l'unité de spécification de type (11) peuvent être modifiés par l'utilisateur par le dispositif d'entrée (15).

5. Appareil de diagnostic par ultrasons selon la revendication 1, comprenant en outre :
un lecteur d'informations de type (31) qui lit des informations de type à partir d'un support d'informations de type fixé à l'objet d'insertion,
dans lequel l'unité de spécification de type (11) spécifie le type de l'objet d'insertion sur la base des informations de type lues par le lecteur d'informations de type (31).

6. Appareil de diagnostic par ultrasons selon la revendication 1, comprenant en outre :
une unité de caméra (41) qui acquiert une image de l'objet d'insertion en imageant l'objet d'insertion ; et
une unité de reconnaissance d'objet d'insertion qui reconnaît l'objet d'insertion sur la base de l'image de l'objet d'insertion acquise par l'unité de caméra (41),
dans lequel l'unité de spécification de type (11) spécifie le type de l'objet d'insertion sur la base de l'objet d'insertion reconnu par l'unité de reconnaissance d'objet d'insertion.

7. Appareil de diagnostic par ultrasons selon la revendication 1, comprenant en outre :
une unité de reconnaissance d'objet d'insertion qui reconnaît l'objet d'insertion sur la base de l'image ultrasonore acquise par l'unité d'imagerie (5),
dans lequel l'unité de spécification de type (11) spécifie le type de l'objet d'insertion sur la base de l'objet d'insertion reconnu par l'unité de reconnaissance d'objet d'insertion.

8. Appareil de diagnostic par ultrasons selon l'une quelconque des revendications 5 à 7, dans lequel les informations standard acquises par l'unité d'acquisition d'informations standard (10) et le type de l'objet d'insertion spécifié par l'unité de spécification de type (11) sont affichées sur le dispositif d'affichage (7).

9. Appareil de diagnostic par ultrasons selon la revendication 8, comprenant en outre :
un dispositif d'entrée permettant á l'utilisateur d'effectuer une opération d'entrée,
dans lequel les informations standard acquises par l'unité d'acquisition d'informations standard (10) et le type de l'objet d'insertion spécifié par l'unité de spécification de type (11) peuvent être modifiés par l'utilisateur par le dispositif d'entrée.

10. Appareil de diagnostic par ultrasons selon l'une quelconque des revendications 1 à 9, dans lequel l'unité de notification (13) met en évidence le vaisseau sanguin déterminé par l'unité de détermination d'insertabilité (12) que l'objet d'insertion peut être inséré, sur le dispositif d'affichage (7).

11. Appareil de diagnostic par ultrasons selon la revendication 10,
dans lequel l'objet d'insertion a un code de couleur attribué en fonction des informations standard, et
l'unité de notification (13) met en évidence le vaisseau sanguin avec la même couleur que le code de couleur de l'objet d'insertion.

12. Appareil de diagnostic par ultrasons selon l'une quelconque des revendications 1 à 11, dans lequel l'image ultrasonore inclut une pluralité de vaisseaux sanguins extraits par l'unité d'extraction de vaisseau sanguin (8),
l'unité de détermination d'insertabilité (12) détermine l'insertabilité de l'objet d'insertion par rapport à chacun de la pluralité de vaisseaux sanguins, et
l'unité de notification (13) affiche, parmi la pluralité de vaisseaux sanguins, le vaisseau sanguin déterminé par l'unité de détermination d'insertabilité (12) que l'objet d'insertion peut être inséré et le vaisseau sanguin déterminé par l'unité de détermination d'insertabilité (12) que l'objet d'insertion ne peut pas être inséré, sur le dispositif d'affichage (7) de manière distincte.

13. Appareil de diagnostic par ultrasons selon la revendication 12, comprenant en outre :
une unité de calcul d'angle d'insertion qui calcule un angle d'insertion de l'objet d'insertion en analysant l'image ultrasonore acquise par l'unité d'imagerie (5),
dans lequel l'unité de détermination d'insertabilité (12) détermine l'insertabilité de l'objet d'insertion par rapport à chacun de la pluralité de vaisseaux sanguins sur la base de l'angle d'insertion calculé par l'unité de calcul d'angle d'insertion, et
l'unité de notification (13) met en évidence, parmi la pluralité de vaisseaux sanguins, le vaisseau sanguin déterminé par l'unité de détermination d'insertabilité (12) que l'objet d'insertion peut être inséré, sur le dispositif d'affichage (7).

14. Appareil de diagnostic par ultrasons selon l'une quelconque des revendications 1 à 13, comprenant en outre :
une mémoire de sauvegarde d'informations standard (9) qui sauvegarde une pluralité de pièces des informations standard,
dans lequel l'unité d'acquisition d'informations standard (10) acquiert une pièce des informations standard parmi la pluralité de pièces d'informations standard sauvegardées dans la mémoire de sauvegarde d'informations standard (9).

15. Appareil de diagnostic par ultrasons selon l'une quelconque des revendications 1 à 13,
dans lequel l'appareil de diagnostic par ultrasons est connecté à un serveur externe via un réseau, et
l'unité d'acquisition d'informations standard (10) acquiert les informations standard via le serveur.

16. Appareil de diagnostic par ultrasons selon la revendication 15, comprenant en outre :
un capteur GPS qui mesure des informations de position de l'appareil de diagnostic par ultrasons,
dans lequel l'unité d'acquisition d'informations standard (10) acquiert les informations standard correspondant aux informations de position mesurées par le capteur GPS.

17. Procédé de contrôle d'un appareil de diagnostic par ultrasons qui affiche un vaisseau sanguin d'un sujet dans lequel un objet d'insertion doit être inséré, sur une image ultrasonore, le procédé de contrôle comprenant :
amener un réseau de transducteurs (2) à transmettre un faisceau ultrasonore vers le sujet, et recevoir un écho ultrasonore du sujet pour acquérir l'image ultrasonore ;
afficher l'image ultrasonore acquise ;
acquérir des informations standard incluant des normes concernant au moins un type d'objet d'insertion vasculaire ;
spécifier un type de l'objet d'insertion que l'utilisateur essaie d'insérer dans le vaisseau sanguin du sujet ;
extraire le vaisseau sanguin en analysant l'image ultrasonore acquise ;
déterminer l'insertabilité de l'objet d'insertion par rapport au vaisseau sanguin extrait sur la base des informations standard acquises et du type spécifié de l'objet d'insertion ; et
notifier d'un résultat de détermination ;
dans lequel l'étape de détermination d'insertabilité de l'objet d'insertion comprend :
déterminer que l'objet d'insertion que l'utilisateur essaie d'insérer dans le sujet ne peut pas être inséré dans le vaisseau sanguin (B) du sujet dans un cas où la longueur de l'objet d'insertion spécifiée par l'unité de spécification de type (11) est acquise en référence aux informations standard (L) et la longueur acquise de l'objet d'insertion est égale ou inférieure à la distance (D) depuis la surface corporelle du sujet jusqu'au vaisseau sanguin (B), qui est mesurée par l'unité d'extraction de vaisseau sanguin (8) ;
déterminer que l'objet d'insertion que l'utilisateur essaie d'insérer dans le sujet peut être inséré dans le vaisseau sanguin (B) du sujet, dans un cas où la longueur de l'objet d'insertion spécifiée par l'unité de spécification de type (11) est acquise en référence aux informations standard (L) et la longueur acquise de l'objet d'insertion est plus longue que la distance (D) depuis la surface corporelle du sujet jusqu'au vaisseau sanguin (B), qui est mesurée par l'unité d'extraction de vaisseau sanguin (8) ;
déterminer que l'objet d'insertion que l'utilisateur essaie d'insérer dans le sujet ne peut pas être inséré dans le vaisseau sanguin (B) du sujet, dans un cas où la longueur de l'objet d'insertion spécifiée par l'unité de spécification de type (11) et un angle d'insertion recommandé de l'objet d'insertion sont acquises en référence aux informations standard (L) et il est estimé que l'objet d'insertion ne peut pas atteindre le vaisseau sanguin (B) à la distance (D) depuis la surface corporelle du sujet, qui est mesurée par l'unité d'extraction de vaisseau sanguin (8) ;
déterminer que l'objet d'insertion que l'utilisateur essaie d'insérer dans le sujet peut être inséré dans le vaisseau sanguin (B) du sujet, dans un cas où la longueur de l'objet d'insertion spécifiée par l'unité de spécification de type (11) et un angle d'insertion recommandé de l'objet d'insertion sont acquises en référence aux informations standard (L) et il est estimé que l'objet d'insertion peut atteindre le vaisseau sanguin (B) à la distance (D) depuis la surface corporelle du sujet, qui est mesurée par l'unité d'extraction de vaisseau sanguin (8) ;
déterminer que l'objet d'insertion que l'utilisateur essaie d'insérer dans le sujet ne peut pas être inséré dans le vaisseau sanguin (B), dans un cas où le diamètre extérieur de l'objet d'insertion spécifié par l'unité de spécification de type est acquis en référence aux informations standard (L) et un rapport du diamètre extérieur de l'objet d'insertion spécifié par l'unité de spécification de type (11) au diamètre (H) du vaisseau sanguin (B) mesuré par l'unité d'extraction de vaisseau sanguin (8) est supérieur à une valeur seuil, même si l'on estime que l'objet d'insertion peut atteindre le vaisseau sanguin (B) ; et
déterminer que l'objet d'insertion que l'utilisateur essaie d'insérer dans le sujet peut être inséré dans le vaisseau sanguin (B), dans un cas où le diamètre extérieur de l'objet d'insertion spécifié par l'unité de spécification de type est acquis en référence aux informations standard (L) et un rapport du diamètre extérieur de l'objet d'insertion spécifié par l'unité de spécification de type (11) au diamètre (H) du vaisseau sanguin (B) mesuré par l'unité d'extraction de vaisseau sanguin (8) est égal ou inférieur à la valeur seuil et il est estimé que l'objet d'insertion peut atteindre le vaisseau sanguin (B).
